# EUROPEAN PATENT APPLICATION

(11) **EP 1 612 223 A2**
(43) Date of publication of application: **04.01.2006**
(21) Application number: 05076908.2
(22) Date of filing: 15.09.1995
(51) Int. Cl.: C07K 14/735, C12N 15/62, A61K 38/17

(54) **Polypeptides with FC binding ability**

(30) Priority: 16.09.1994 AU PM823294; 31.10.1994 US 332562
(62) Divisional of application: 95932563.0
(71) Applicant: THE AUSTIN RESEARCH INSTITUTE, Heidelburg, Victoria 3084 (AU)
(72) Inventor: The designation of the inventor has not yet been filed
(74) Representative: Arends, William Gerrit

(57) **Abstract**

An isolated polypeptide with Fc binding ability. The polypeptide comprises an Fc binding component comprising an extracellular region of a native FcγRII receptor and a fusion component.

## Description

### Field of Invention

The present invention generally relates to molecules having Fc binding ability such as those with Fc receptor-like activity. The present invention also relates to the molecules, nucleic acids encoding the molecules, antagonist compounds, pharmaceutical compositions comprising the molecules and compounds, methods for testing potential antagonists, methods for producing the polypeptides, methods of treatment of disease and other aspects.

### Background of the invention

Cell surface receptors for the Fc portion of IgG (Fc_{γ}R) are expressed on most hematopoietic cells, and through the binding of IgG play a key role in homeostasis of the immune system and host protection against infection. By way of example Fc_{γ}RII is a low affinity receptor for IgG that essentially binds only IgG immune complexes and IgA immune complexes and is expressed on a diverse range of cells such as monocytes, macrophages, neutrophils, eosinophils, platelets and B cells (1-3). Fc_{γ}RII is involved in a number of immune responses including antibody-dependent cell-mediated cytotoxicity, clearance of immune complexes, release of inflammatory mediators and regulation of antibody production (1-6).

Similarly Fc receptors for other classes of immunoglobulin also occur. For example the Fc receptor for IgE is present on mast cells, basophils and Langerhans cells.

Both the IgG and the IgE Fc receptors contain an extracellular Ig-interactive region which comprises two Ig-like disulphide bonded extracellular domains of the C2 set (7-11). These receptors are structurally conserved in all the Ig-superfamily leukocyte FcR (including Fc_{γ}RI, Fc_{γ}RIII, Fc_{ε}RI and FcαRI) and presumably represents an Ig-interactive motif (12-16). In previous studies the inventors identified the IgG binding region of human Fc_{γ}RII (17, 18). Chimeric Fc_{γ}RII/Fc_{ε}RI α chain receptors were used to demonstrate that the second extracellular domain of Fc_{γ}RII was responsible for the binding of IgG, with a direct binding region located between residues Asn¹⁵⁴ to Ser¹⁶¹. Molecular modelling of Fc_{γ}RII domain 2 predicted a structure comprising 7 β strands (A, B, C, C', E, F, G) forming two antiparallel β sheets (containing the ACFG and BC'E strands respectively), stabilised by a disulphide bond between strands B and F and a core of hydrophobic residues (20). The Asn¹⁵⁴ to Ser¹⁶¹ binding region was shown to encompass an exposed loop region (the F-G loop) at the interface of domains 1 and 2.

In work leading up to the present invention, the inventors surprisingly discovered that alteration of amino acid residues in the Fc receptors lead to altered affinities for immunoglobulin.

### Summary of the Invention

The invention relates to a polypeptide with Fc binding ability wherein the polypeptide is altered compared to a native Fc receptor by addition, deletion or substitution of one or more amino acids compared to said native Fc receptor.

The invention also relates to a method of testing compounds for their ability to act as an Fc receptor antagonist, to the antagonist compounds identified by the method, to nucleic acid molecules encoding the polypeptides of the invention and to methods of making the nucleic acid molecules. In addition the invention relates to methods of detecting immunoglobulin, methods of removing immunoglobulin, methods of treatment and pharmaceutical compositions involving the peptides of the invention or their antagonists.

### Brief Description of the Figures

Figure 1. IgG complex binding of chimeric Fc receptors. COS-7 cell monolayers were transfected with chimeric cDNA constructs: D1εD2γ (a), γ109-116ε (b), γ130-135ε (c), or Fc_{ε}RI (d). The binding of IgG immune complexes was assessed directly on the monolayers by EA rosetting using mouse IgG1 sensitised erythrocytes.
Figure 2. Human IgG1-dimer binding of chimeric Fc receptors. Radiolabelled dimeric human IgG1 was titrated on COS-7 cells transfected with wild-type Fc_{γ}RIIa (■) or chimeric receptor cDNAs; D1εD2γ (□), γ109-116ε (●), γ130-135ε (○). All of the chimeras were expressed on the cell surface as determined by EA rosetting outlined in Figure 1.

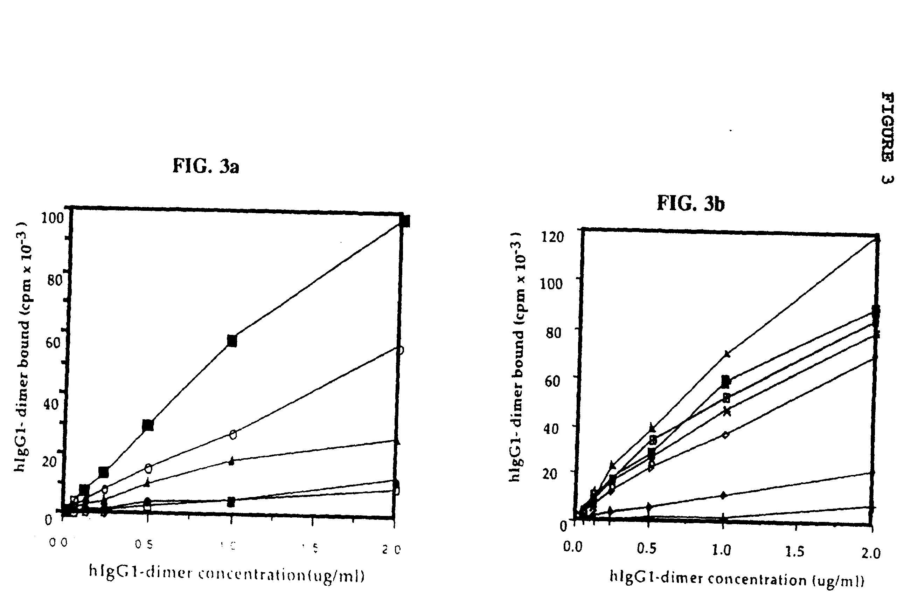

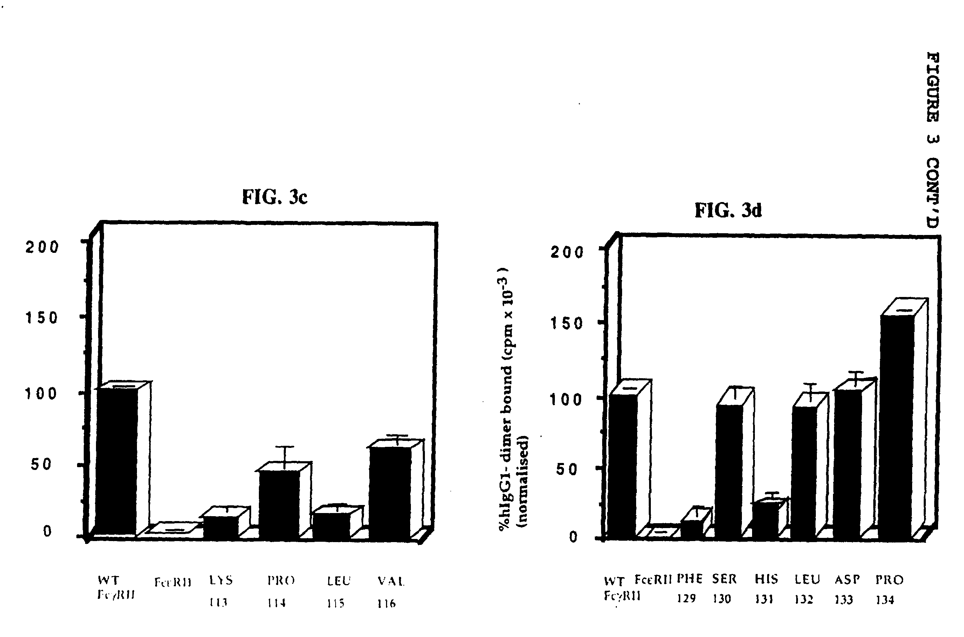

Figure 3. Human IgG1-dimer binding by Fc_{γ}RIIa alanine point mutants. Radiolabelled dimeric human IgG1 was titrated on COS-7 cells transfected with wild-type Fc_{γ}RIIa or Fc_{γ}RIIa containing alanine point mutations, (A) B-C loop mutants, Lys¹¹³-Ala (□), Pro¹¹⁴-Ala (▲), Leu¹¹⁵-Ala (●), Val¹¹⁶-Ala (○), (B) C'-E loop mutants, Phe¹²⁹-Ala (+), Ser¹³⁰-Ala (◇), Arg/His¹³¹-Ala (◆), Leu¹³²-Ala (X), Asp¹³³-Ala ( 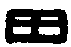 ), Pro¹³⁴-Ala (△). Comparison of the levels of human IgG1 dimer binding to Fc_{γ}RII mutants relative to wild-type Fc_{γ}RIIa, (C) B-C loop mutants, (D) C'-E loop mutants. The binding of wild-type Fc_{γ}RIIa taken as 100% and mock transfected cells as 0% binding. Results are expressed as + S.E. To control for variable receptor expression between the mutant Fc_{γ}RII COS-7 cell transfectants, levels of expression were determined using a radiolabelled monoclonal anti-Fc_{γ}RII antibody 8.2, and dimer binding normalised to that seen for wild-type Fc_{γ}RII. Typical levels of 8.2 binding in cpm + S.E : WT Fc_{γ}RII 95279; Lys¹¹³-Ala 71660; Val¹¹⁴-Ala 61636; Leu¹¹⁵-Ala 44696; Pro¹¹⁶-Ala; Phe¹²⁹-Ala 74707; Ser¹³⁰-Ala 139802; Arg/His¹³¹-Ala 140475; Leu¹³²-Ala 121096; Asp¹³³-Ala 100149; Pro¹³⁴-Ala 172047.
Figure 4. Molecular model of the extracellular Ig interactive region of Fc_{γ}RII putatively involved in the interaction with IgG1. The position of the loops and G/A strand from domains 1 and 2 are indicated. Examples of amino acids, mutations of which alter Fc receptor function such as Phe¹⁶⁰ and Gly¹⁵⁶ are also shown.
Figure 5. Oligonucleotides used in SOE-PCR of Example 2.
Figure 6. Histogram showing the effect of mutations on IgE receptor binding immunoglobulin.
Figure 7. Histogram showing a comparison of Fc receptor mutants binding IgG₁ and IgG₂.
Figure 8. Graph showing efficiency with which chimeric receptors bind IgE, εεγ ( ), γεγ ( ), CC' ( ), EF ( ) and GC ( ).
Figure 9. Photograph of SDS-PAGE showing specificity of the fusion protein, (HSA:FcγRII) for mouse IgG2a (γ2a) IgrG2b (γ2b) and HAGG but not for Fab'2 fragments (1302). Shows epitopes present in the fusion protein as detected by four different anti-FcγRII antibodies (8.2, 8.26, IV-3 & 8.7). The fusion protein was radiolabelled with I¹²⁵ and precipitated using Ig or antibodies shown then subjected to SDS-PAGE after reduction.
Figure 10a. Graph showing the clearance of HSA:FcγRII ( 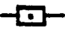 ) and FcγRII (―●―) in the blood.
Figure 10b. Graph showing failure of HSA:FcγRII to accumulate in the urine affinities.
Figure 11. Graph showing the binding affinities of HAGG to HSA:FcγRII-silica (●) and HSA silica (○). HAGG did not bind HSA:silica.
Figure 12. Nucleotide and predicted amino acid sequence of HSA: Fc_{γ}RII DNA.
Figure 13. Graphs showing results of ELISA studies on the ability of the fusion protein, rsHSA-FcR (―●―) to bind immune complexes. The recombinant receptor rsFcR is denoted by ( 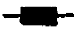 ).
Figure 14a. ELISA studies of serum from a patient with rheumatoid arthritis. Plates were coated with anti-Fc_{γ}RII antibody and then with rec.sFc_{γ}RII.
Figure 14b. Shows results of plates coated with rec.sFc_{γ}RII.
Figure 15. Graph of heat depleted HAGG using Fc_{γ}RII-HSA silica (●) This shows that immune complexes are depleted from liquid by incubation with HSA:Fc_{γ}RII protein silica resin but not by HSA-silica resin (○) no silica is denoted by (■).
Figure 16. Graph showing no depletion of monomeric immunoglobulin using Fc_{γ}RII-HSA silica. This indicates the fusion protein does not bind monomeric Ig.

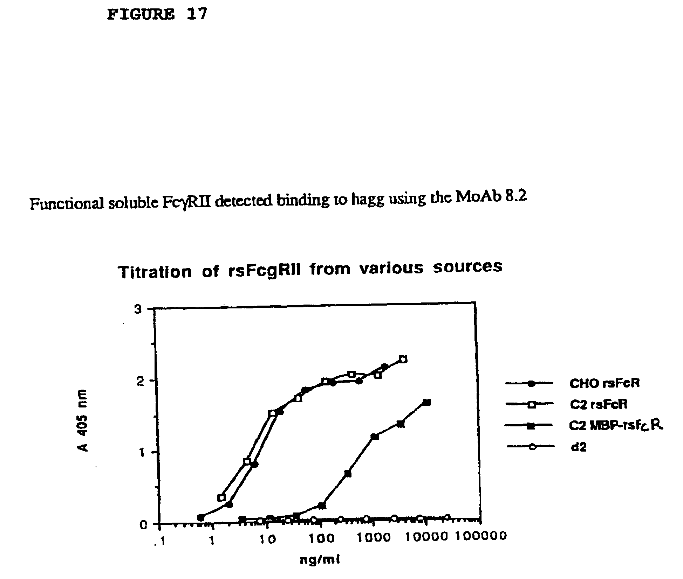

Figure 17. Titration of rsFc_{γ}RII from various sources. The binding of the recombinant protein from various sources is detected by use of anti-FcRII antibody 8.2 followed by anti-mouse Ig labelled with peroxidase. The sources of the recombinant protein are CHO cells (CHO rs FcR) (―●—), bacteria expressing a fusion protein consisting of the extracellular domains of Fc_{γ}RII fused to maltose binding protein (C2 MBP-rsFcR) (―■―), the extracellular domains of Fc_{γ}RII cleaved from the Fc_{γ}RII maltose binding fusion protein (C2 rs FcR) (―□―). A fusion protein (d2) 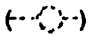 was used as a control. This contains a single FcR domain and has no functional activity.
Figure 18. Graph of inhibition of HRP labelled rsHSA-Fc_{γ}RII by rheumatoid factors and sera. Titration of patients' sera in the HRP-HSA:Fc_{γ}RII ELISA assay. Patients' sera (columns 1-14) was titrated on Hagg coated plates prior to addition of the HRP fusion protein conjugate. Titration of normal serum is also shown (column 15). No inhibition of activity was seen with normal serum but, all sera except column 12 profoundly inhibited Fc receptor binding to Hagg.
Figure 19. Names of various peptiods and absorbences thereof in the test for antagonist compounds.

### Detailed Description of Preferred Embodiments

The invention relates to a polypeptide with Fc binding ability wherein the polypeptide is altered compared to a native Fc receptor by addition, deletion or substitution of one or more amino acids such that said alteration results in improved characteristics compared to said nature receptor.

The term "a polypeptide with Fc binding ability" means a polypeptide or protein comprising natural or synthetic amino acids which has an ability to bind the Fc region of immunoglobulin. The immunoglobulin may be of any class such as IgG, IgE, IgA, IgM or IgD. The immunoglobulin may be derived from any animal such as human, rat, mice, cattle, sheep, goats and other domestic animals, including birds such as chickens, ostriches and emus.

The term "altered compared to a native Fc receptor" means that the polypeptide is different to the native Fc receptor. Such difference may include differences in immunoglobulin binding ability, difference in therapeutic ability , amino acid composition or solubility and the like.

The term "improved characteristics" means that a desirable characteristic compared to the native Fc receptor is achieved. This characteristic may enhance or decrease Fc binding ability, cause increased serum half life of the polypeptide for use as a therapeutic or make the polypeptide detectable.

In a first embodiment the present invention relates to a polypeptide with Fc binding ability wherein the polypeptide is an Fc receptor-like molecule with an altered ability to bind immunoglobulin wherein said altered ability is brought about by alteration of one or more amino acid residues which affect immunoglobulin binding.

The phrase "Fc receptor-like molecule" means a molecule which is able to bind immunoglobulin to at least some degree. The immunoglobulin may be IgG, IgE, IgA, IgM or IgD. The molecule will usually be a peptide, polypeptide or protein, or made up, at least partially, of amino acids. In its most usual form the molecule will be a peptide composed of a number of amino acid residues linked by peptide bonds. The amino acid components may be natural or synthetic and will be known to those skilled in the art.

The phrase "an altered ability to bind immunoglobulin" means that the molecule has an immunoglobulin binding activity different to that of one or more native Fc receptors. This includes the ability of the molecule to bind one form of immunoglobulin compared to another form of immunoglobulin i.e. where the molecule has an altered ability to bind immune complexes, aggregates, dimeric or monomeric immunoglobulin compared to a native Fc receptor. The activity of the molecule may be increased or decreased compared to a native Fc receptor for a given immunoglobulin class.

The phrase "alteration of one or more amino acid residues which affect immunoglobulin binding ability" means that the comparable amino acid residue or region of amino acid residues, implicated in immunoglobulin binding in a native Fc receptor are changed in the Fc receptor-like molecule. The amino acids implicated in immunoglobulin binding may function directly in immunoglobulin binding or may be involved indirectly such as by maintaining the structural integrity of the receptor so that binding can occur. The change(s) may be the result of insertion, deletion or substitution.

The present inventors have determined that amino acid residues or regions of residues in the first and second domains of the Fc_{γ}RII receptor and Fc_{εR}I receptor function in binding of immunoglobulin. As the extracellular regions of Fc receptors for immunoglobulins are conserved it is expected that similar regions of Fc receptors for other immunoglobulins such as IgA, IgM and IgD will be implicated in immonoglobulin binding and hence be within the ambit of the present invention.

Preferably the Fc receptor-like molecule is in the form of an isolated preparation meaning it has undergone some purification away from other proteins and/or non-proteinaceous molecules. The purity of the preparation may be represented at least 40% Fc receptor-like molecule, preferably at least 60% Fc receptor-like molecule, more preferably at least 75% Fc receptor-like molecule, even more preferably at least 85% Fc receptor-like molecule and still more preferably at least 95% Fc receptor-like molecule relative to non-Fc receptor-like molecule material as determined by weight, activity, amino acid similarity, antibody reactivity or any other convenient means.

The Fc receptor-like molecule may be bound to a cell membrane or a support means, or be soluble in form. Where a pharmaceutical use is indicated preferably the molecule is soluble for example as a genetically engineered soluble molecule.

Soluble Fc receptor-like molecules can be made by methods such as those of Ierino *et al* J. Exp. Mod. (Nov) 1993.

The molecule may also be labelled with a reporter molecule providing, under suitable conditions, a detectable signal. Such reporter molecules include radio-nucleotides, chemiluminscent molecules, bioluminescent molecules, fluorescent molecules or enzymes. Commonly used enzymes include horseradish peroxidase, glucose oxidase, β-glactosidase and alkaline phosphatase amongst others.

Preferably the Fc receptor-like molecule is an amino acid mutant, variant or derivative of a native Fc receptor. This means that the Fc receptor-like molecule comprises a peptide which has undergone deletion, insertion, substitution or other changes to its amino acid residues compared to a native Fc receptor. The native Fc receptor providing the basis for the mutant, variant or derivative may be derived from human or animal species. Such animal species are preferably mammalian species such as mice, rats, rabbits, bovine, ovine, porcine or caprine species.

Deletion, insertion or substitution of amino acids to produce the Fc receptor-like molecule of the present invention may be performed by known means. Where the Fc receptor-like molecule is recombinant derived, the nucleic acid encoding the molecule will have incorporated in its sequence the appropriate code for one or more amino acid insertions or substitutions or have undergone the appropriate deletion from its coding sequence. Where the receptor-like molecule is produced by *de novo* peptide synthesis the desired amino acid sequences may be incorporated.

Isertions or substitutions may be achieved by placing a stretch of amino acid residues from another type of Fc receptor into the Fc receptor-like molecule which is being constructed. For example the first domain from one receptor such as Fc_{ε} receptor may be used to replace the first domain in Fc_{γ} receptor to produce the desired result. Alternatively, or in addition, site directed mutagenesis or other techniques may be used to achieve amino acid substitution. Deletions may be achieved by removal of one or more amino acids.

Substitution of amino acids may be conservative by replacing an amino acid with a residue of similar properties. For example, the amino acid substitution may be in accordance with Table 1.

**Table 1 Suitable residues for amino acid substitutions**

| Original Residue | Exemplary Substitutions |
|---|---|
| Ala | Ser |
| Arg | Lys |
| Asn | Gln; His |
| Asp | Glu |
| Cys | Ser |
| Gln | Asn |
| Glu | Ala |
| Gly | Pro |
| His | Asn; Gln |
| Ile | Leu; Val |
| Leu | Ile; Val |
| Lys | Arg; Gln; Glu |
| Met | Leu; Ile |
| Phe | Met; Leu; Tyr |
| Ser | Thr |
| Thr | Ser |
| Trp | Tyr |
| Tyr | Trp; Phe |
| Val | Ile; Leu |

Alternatively substitutions may be with an amino acid of different chemical characteristics imparting a different character to the molecule when compared to a native Fc receptor.

In a preferred embodiment the invention relates to an Fc receptor-like molecule having enhanced ability to bind immunoglobulin wherein the enhanced ability is brought about by alteration of one or more amino acid residues which affect immunoglobulin binding ability.

The phrase "enhanced ability to bind immunoglobulin" means that the molecule has an immunoglobulin binding activity higher than, or increased compared to, a native Fc receptor in a given class.

The alteration of one or more amino acid residues may be in the first or second domain.

Where the Fc receptor-like molecule has an enhanced ability to bind IgG, preferably alterations in the first domain to included changes to the A/B, C/C' and/or E/F loops and/or G/A strand. The loops referred to hereafter are the loops identified in the putative 3-D structures or their equivalents for the receptors discussed earlier and identified in the Examples. The term "equivalents" means amino acid residues that occur in the same position on the native Fc receptor which comprise the putative loops. It also includes eqivalent loop structures in other native Fc receptors. In addition all of the amino acid residue positions discussed herein are relative to the amino acid sequences of Fc_{γ}RII or Fc_{ε}RI.

The loops of F_{cγ}RII are as follows:

| | | |
|---|---|---|
| Domain 1 | A/B | Glu¹⁰ - Ser²¹ |
| | C/C' | Asn⁴² - Ile⁴⁶ |
| | B/F | Asn⁵⁹ - Asp⁶² |
| Domain 2 | B/C | Ser¹⁰⁹ - Val¹¹⁶ |
| | C'/E | Phe¹²⁹ - Pro¹³⁴ |
| | F/G | Asn¹⁵⁴ - Ser¹⁶¹ |
| G/A strand | | Val⁷⁹ - Pro⁹³ |

Loops for FRI are:

| | | |
|---|---|---|
| Domain 1 | A/B | Asn¹⁰ - Asn²¹ |
| | C/C' | Asn⁴² - Leu⁴⁵ |
| | E/F | Lys⁵⁹ - Glu⁶¹ |
| Domain 2 | B/C | Trp¹¹⁰ - Lys¹¹⁷ |
| | C'/E | Tyr¹²⁹ - His¹³⁴ |
| | F/G | Lys¹⁵⁴ - Ile¹⁶⁹ |
| G/A strand | | Val⁷⁹ - Ser⁹³ |

Alterations to the second domain of a Fc receptor-like molecule specific for IgG include changes in the B/C, C'/E and/or F/G loops, and/or Q/A strand which connects domains 1 and 2.

Preferably the changes comprise substitution of one or more amino acids especially a conservative substitution. Such changes include but are not limited to replacement by alanine, glycine, serine, asparagine or small synthetic neutrally charged amino acids. Most preferably the replacement is alanine.

More preferably the alterations are at the following positions 133, 134, 158, 159 and/or 160.

Still more preferably the Asp¹³³ and/or Pro¹³⁴ residues of FcγRII are replaced by alanine.

Where the Fc receptor-like molecule has an enhanced ability to bind IgE preferably the alterations in the first domain include changes in A/B, C/C' and/or B/F loops and/or the G/A strand that connects domain 1 and domain 2.

Alterations to the second domain of a Fc receptor-like molecule specific for IgE include changes in the B/C, C'/E and/or F/G loops.

More preferably the changes are at the following positions 130, 156, 160 and/or 161.

Still more preferably the Trp¹³⁰, Trp¹⁵⁶, Tyr¹⁶⁰ and/or Glu¹⁶¹ is/are replaced by alanine.

In another preferred embodiment the invention relates to an Fc receptor-like molecule having reduced ability to bind immunoglobulin wherein the reduced ability is brought about by the alteration of one or more amino acid residues which affect immunoglobulin binding ability.

The phrase "reduced ability to bind immunoglobulin" means that the molecule has an immunoglobulin binding activity lower than, or decreased compared to, a native Fc receptor in a given class. This includes a reduced activity for binding of one form of immunoglobulin such as, for example, dimeric immunoglobulin.

The reduced binding ability may be brought about by deletions, insertions or substitutions of amino acid residues in the first or the second domain. Preferably the reduced binding ability will be the result of substitution or deletion of one or more amino acid residues although insertions are also clearly contemplated.

Preferably substitutions will be with an amino acid residue (natural or synthetic) which has different chemical characteristics to the corresponding amino acid in the relevant native Fc receptor in question. Such as for example the substituted amino acid may have different charge, acidity or basicity.

The additions, substitutions and/or deletions may be made in accordance with standard methods as described above.

Where Fc receptor-like molecule has a reduced ability to bind IgG preferably alterations in the first domain include replacement of that domain or changes to the A/B, C/C' or E/F loops or G/A strand.

More preferably the changes are at the following positions 10 to 21, 42 to 48 and/or 59 to 62.

Alterations to the second domain of a Fc receptor-like molecule specific for IgG preferably include changes to the F/G, B/C, or C'/C loops.

More preferably the changes are at the following positions 113, 114, 115, 116, 129, 131, 155 and/or 156.

Still more preferably the first domain is deleted or replaced by a domain from an Fc receptor for another immunoglobulin.

Alternatively still more preferably the Lys¹¹³, Pro¹¹⁴, Leu¹¹⁵, Val¹¹⁶, Phe¹²⁹ and/or Arg/His¹³¹ of Fc_{γ}RII is/are replaced by alanine.

Where the Fc receptor-like molecule has a reduced ability to bind IgE preferably alterations in the first domain include the A/B, C/C' or B/F loops.

More preferably the changes are at the following positions in the first domain 10 to 21, 42 to 48 and/or 59 to 62.

Alterations in the second domain of a Fc receptor-like molecule specific for IgE preferably include changes to the F/G, C'/B or B/C loops.

More preferably changes are at one or more of the following positions: 131, 132, 155, 158 and/or 159.

Still more preferably the B/C loop (Ser¹⁰⁹ to Val¹¹⁶) of Fc_{ε}RI is deleted or replaced by a B-C loop from a receptor for another immunoglobulin.

Alternatively still more preferably the C'/B loop (Ser¹³⁰ to Thr¹³⁵) of Fc_{ε}RI is deleted or replaced by a C' /E loop from a receptor from another immunoglobulin.

Still more preferably Tyr¹³¹, Glu¹³², Val¹⁵⁵, Leu¹⁵⁶ and/or Asp¹⁵⁹ of Fc_{ε}RI is/are replaced by alanine.

In addition to the alteration discussed earlier the alterations contemplated may be other alterations which make the polypeptide useful as a therapeutic or reagent Thus the alteration may be in the form of an addition deletion or subtraction. For example where addition is contemplated a polypeptide or other suitable molecule may be added to a native Fc receptor in order to increase the size of the molecule or to provide a linker which links a native Fc receptor with a reporter molecule.

In a second embodiment the invention relates to a polypeptide with Fc binding ability wherein the polypeptide is altered compared to a native Fc receptor such that the size of the polypeptide is larger than said native Fc receptor.

The inventors have surprisingly found that the addition of an amino acid sequence to a polypeptide with Fc binding ability not only results in an extended life in the body of an animal but also retains biological activity of the Fc binding component. Thus, the augmented polypeptide has a greater serum half life compared to soluble protein with Fc binding ability and is therefore capable of being more effective as a therapeutic than an altered soluble native Fc receptor.

Preferably the polypeptide with Fc binding ability is in an isolated form such as that described earlier in relation to the Fc receptor-like molecule.

Preferably the polypeptide of the invention is in soluble form where it is to be used in serum or other administration routes requiring solubility. This would generally mean that the transmembrane region is not included however the intracellular region may be included.

Preferably the augmentation of the Fc binding polypeptide is achieved by linking an amino acid sequence, such as a second polypeptide or other suitable molecule to a peptide with Fc binding ability. The peptide with Fc binding ability may be a native receptor, a modified native receptor (for example a receptor without the transmembrane or cytoplasmic regions) or a Fc receptor-like molecule as described earlier.

Preferably the polypeptide with Fc binding ability is of a size larger than about 67 kD since proteins below this size are excreted by the kidneys. More preferably the polypeptide is in the size range of 67 to 1000 kD. Still more preferably the polypeptide is approximately 100 kD.

Preferably the augmentation is achieved by adding a peptide, polypeptide or other molecule which is well tolerated in an animal. Such peptides or polypeptides include human serum albumin (HSA), bovine serum albumin, other Fc receptors, immunoglobulin from any species, cytokines, complement regulating molecules (eg. CD46, CD55, CD59), complement receptors and cytokine receptors. Such additions could include more than one molecule of the same or a different type. The other molecules suitable include dextrans, carbohydrates, polyethylene glycoland synthetic polymers.

The polypeptide may be directed against any class of Ig. Preferably the polypeptide is directed against IgG or IgE. Even more preferably the polypeptide is HSA:FcγRII as herein described.

The polypeptide with Fc binding ability may be produced by any convenient means such as through recombinant DNA technology as a fusion protein, or alternatively the two components may be produced separately (by recombinant DNA or other means) and then linked. Alternatively one or both components may be made via peptide synthesis. These methods are described in more detail later on.

In a third embodiment the invention relates to a polypeptide with Fc binding ability comprising a component capable of detection.

The term "component capable of detection" means that the polypeptide is linked to or contains a detectable signal such as a reporter molecule, a biosensor or a molecule which may be directly or indirectly detected. The reporter or label is a component which is capable of detection such as by radio labelling, chemiluminescent labelling, fluorometric labelling, chromophoric labelling or labelled antibody binding. Detection may be achieved by direct labelling of the polypeptide with a ligand such as, for example, biotin which specifically binds to streptavidin linked covalently to horseradish peroxidase or another enzyme such as alkaline phosphatase. The actual component capable of detection may be suitably chosen by those skilled in the art.

Preferably the polypeptide is in an isolated form as described above.

Preferably the polypeptide comprising a component capable of detection comprises the polypeptide described in the first or second embodiments of the invention. Preferably the component capable of detection is present on, or comprises part of the augmentation. Alternatively, the component capable of detection may be present on the Fc binding portion of the molecule provided this does not inhibit Fc binding ability.

Preferably the component capable of detection is an enzyme such as horseradish peroxidase.

In another embodiment the present invention relates to a method of testing a compound for its ability act as an antagonist of an Fc receptor said method comprising contacting the compound with a polypeptide with Fc binding ability or a native Fc receptor under conditions and for a time sufficient to allow binding of the compound to the polypeptide or receptor and determining whether binding has occurred.

The term "Fc receptor" used directly above includes any native or non-native Fc receptor or a portion thereof which binds Fc.

The compound tested may be any compound which could possibly be useful as an Fc receptor antagonist. Such compounds may be antibodies, including polyclonal and monoclonal antibodies, or fragments of antibodies, such as scantibodies, antibody mimetics (Smythe & von Itzstein) and the like. The compounds may be extracts from produced from plants or animals such as rain forest plants, corals and the like. The compounds may be peptides or peptide-like substances or other organic substances such as those derived from combinational libraries (Geysen et al 1995; Stratton-Thomas et al 1995, Lorne Conference on Protein Structure and Function, Australia).

The method of the invention may be conducted in any suitable manner known to those skilled in the art. The polypeptide with Fc binding ability or the Fc receptor may be attached to a support leaving the Fc binding site free. Then the immunoglobulin and compound under investigation may be added to the attached polypeptide or Fc receptor. Alternatively Ig or the Fc fragment thereof may be attached to a support. The polypeptide or the Fc receptor and compound under investigation may be added to the bound ligand.

Those skilled in the art will also be familiar with the conditions and time needed to allow any binding of the polypeptide or native Fc receptor to the compound being tested.

Determination of whether binding has taken place may be made by any convenient means. This may be achieved by common detection method such as by using a labelled polypeptide or labelled FcR or by using a labelled Ig. Such detection methods are well known by those skilled in the art and are discussed elsewhere in this document.

The method may be used to screen compounds which are potential inhibitors of receptors for any class of imunoglobulin. Preferably the method is used to screen compounds for their ability to block binding of Ig to the Fcγ receptor or the Fcε receptor.

In another embodiment the present invention relates to antagonist compounds identified by the above method which interfere with the amino acid residues in Fc receptors which are involved in immunoglobulin binding. Such compounds embrace any compound which interact with these amino acid residues or regions of residues so as to interfere with or reduce immunoglobulin binding and include compounds which bind to these residues or regions of residues by hydrophobic, electrostatic or other chemical interaction. This also includes compounds which interfere with the structural integrity of the molecule thereby reducing its affinity for immunoglobulin as well as compounds which directly interfere with the amino acids involved in immunoglobulin binding. Also included are compounds that bind to Ig, as opposed to its receptor, and thereby inhibit the receptor-Ig interaction. The antagonists may be antibodies, peptides, peptide-like substances or any other compound as described above. Preferably the antagonist once identified, is in an isolated form. As such, the Fc receptor antagonists may be used in the treatment of asthma, rheumatoid arthritis, lupus , glomerulonephritis, IgA naphropathies, etc. and a host of immune complex and other diseases including but not limited to autoimmune diseases.

Where the antagonist is intended to block or reduce IgG binding then the compound will preferably interact with the A/B, C/C' or E/F loops in the first domain or with the F/G, B/C or C'/B loops in the second domain or the G/A strand. Preferably the compounds will be capable of binding to or blocking the function of one or more of the following residues in the native Fc receptors 10-21, 42-48, 59-62, 113, 114, 115, 116, 129 131, 133, 156, 158, 159 and/or 160 or their functional equivalents.

Where the antagonist is intended to block or reduce IgE binding in disease such as asthma or allergy then the compound will preferably interact with the A/B, C/C' or E/F loops in the first domain and/or the F/G, C'/B or B/C loops of the second domain or the G/A strand.

Preferably the compounds will be capable of binding to or blocking the function of the following residues: 10-21, 42-48, 59-62, 131, 132, 155, 158 and/or 159.

In another embodiment the present invention contemplates pharmaceutical compositions containing as an active ingredient the polypeptide with Fc binding ability including the Fc receptor-like molecules or the antagonist compounds described above, depending on the condition to be treated, together with a pharmaceutically appropriate carrier or diluent. For example, polypeptide with Fc binding ability or Fc receptor-like molecule with enhanced immunoglobulin binding ability may be used to treat diseases including by not limited to glomerulonephritis, lupus, arthritis, heparin induced thrombocytopoenia thrombosis syndrome (HITTS) or idiopathic thrombocytopoenia pupuera (ITP), asthma, allergy, eczema, Ig nephropathies and rheumatoid arthritis. Fc receptor-like molecule with reduced binding ability may be used to treat disease where it is desirable to remove only some or one particular kind of immunoglobulin. Antagonist compounds may be used in the treatment of inappropriate or excessive immunoglobulin levels or aggregates or immune complexes are part of the symptoms of the disease such as asthma, allergy, rheumatoid arthritis, etc. For the purpose of describing the pharmaceutical compositions, all of the above molecules and compounds will be referred to herein as "active molecules". The use of the term "active molecules" therefore should be read as one or more of the above molecules depending on the condition to be treated.

The active molecules of the pharmaceutical compositions are contemplated to exhibit therapeutic activity when administered in an amount which depends on the particular case. The variation depends, for example, on the animal and the active molecule. For example, from about 0.05 µg to about 100 mg of Fc receptor-like molecule or antagonist compound may be administered per kilogram of body weight per day to alter Fc receptor-immunoglobulin interaction. Dosages may be adjusted to provide the Optimum therapeutic response. For example, several divided doses may be administered daily, weekly, monthly, or in other suitable time intervals or the dose may be proportionally reduced as indicated by the exigencies of the situation. The active molecules may be administered in a convenient manner such as by the oral, intravenous (where water soluble), intraperitoneal, intramuscular, subcutaneous, topical, intranasal, intradermal or suppository routes or implanting (e.g. using slow release molecules). Depending on the route of administration, the active molecules may be required to be coated in a material to protect said molecules from the action of enzymes, acids and other natural condition which may inactivate said ingredients.

The active molecules may also be administered in dispersions prepared in glycerol, liquid polyethylene glycol, and/or mixtures thereof and in oils. Under ordinary conditions of storage and use, these preparations contain a preservative to prevent the growth of microorganisms.

In another embodiment the present invention relates to a nucleic acid molecule encoding a polypeptide with Fc binding ability wherein the structure of the polypeptide is altered compared to a native Fc receptor by addition, deletion and/or substitution of the amino acids encoded by the nucleic acid such that said alteration results in improved characteristics compared to said native receptor.

The term "nucleic acid molecule" refers to molecule made up of natural or synthetic purines and pyrimidines. The nucleic acid molecule may be DNA or RNA, single or double stranded, linear or circular and may form a part of a larger nucleic acid molecule.

The term "polypeptide with Fc binding ability" the meaning given earlier.

The terms "the polypeptide is altered compared to a native Fc receptor" and "improved characteristics" have the same meaning as given earlier.

Preferably the nucleic acid molecule is in isolated form meaning it has undergone some purification away from other nucleic acids and/or non-nucleic acid molecules. The purity of the preparation may be represented by at least 40% nucleic acid molecule encoding a polypeptide with Fc binding ability, preferably at least 60% nucleic acid molecule, more preferably at least 75% nucleic acid molecule, even more preferably at least 85% nucleic acid molecule, even more preferably at leat 95% nucleic acid molecule relative to nucleic acid molecules not encoding a polypeptide with Fc binding ability as determined by nucleic acid homology, sequence or any other convenient means.

In a preferred embodiment the present invention relates to a nucleic acid molecule encoding an Fc receptor-like molecule comprising an altered ability to bind Fc wherein said ability is brought about by alteration of one or more amino acid residues which affect immunoglobulin binding ability.

The phrase "Fc receptor-like molecule" has the meaning given earlier.

The phrases "altered ability to bind immunoglobulin", "the alteration of one or more amino acid residues which affect immunoglobulin binding ability" have the same meanings as given earlier.

The nucleic acid molecule may encode an Fc receptor-like molecule which functions as a receptor for any type of immunoglobulin such as IgG, IgE, IgA, IgM, or IgD. The Fc receptor-like molecule encoded may be derived from any species, such as human, mouse, rat, bovine, ovine, caprine etc and may comprise a combination of different sources. The term "derived from" means that the original coding sequence providing the basis for the nucleic acid molecule prior to alteration comes from the species specified. Those skilled in the art will know which techniques may be used to alter the amino acids encoded by a nucleic acid in order to produce a nucleic acid molecule in accordance with the invention. The nucleic acid molecule may be made by site mutagenesis of DNA encoding native Fc receptor, splice extension overlap PCR, *de novo* synthesis, etc.

Preferably the nucleic acid molecule encodes a mutant, derivative or variant of a native Fc receptor as described earlier.

Preferably the nucleic acid molecule encodes an Fc receptor like peptide which has enhanced ability to bind IgG or IgE. The phrase "enhanced ability" has the same meaning as given earlier.

More preferably where the nucleic acid molecule encodes an Fc receptor-like molecule with an enhanced ability to bind IgG the molecule comprises codons resulting in one or more altered amino acids in the A/B, C/C' and/or E/F loops of the first domain or B/C, C'/E and/or F/G loops of the second domain and/or the G/A strand that connects the two domains.

Still more preferably the codons result in altered amino acids at positions 158, 159, 160, 133 and/or 134. Most preferably the altered amino acids comprise alanine, glycine, serine, asparagine or small synthetic neutrally charged amino acids. Most preferably the codon specifies the amino acid alanine.

Still more preferably the nucleic acid molecule comprises a cDNA encoding Fc_{γ}RII with the codon for Asp¹³³ and/or Pro¹³⁴ specifying alanine. Even more preferable the nucleic acid molecule is Asp¹³³-Ala or Pro¹³⁴-Ala as described in the Examples.

Alternatively more preferably the nucleic acid molecule encodes an Fc receptor-like molecule with an enhanced ability to bind IgE. The molecule comprises codons resulting in one or more altered amino acids in the A/B, C/C' and/or E/F loops of the first domain or the F/G, C'/E and/or B/C loops of the second domain or the G/A strand.

Still more preferably the codons result in altered amino acids at positions 130, 156, 160 and/or 161.

Still more preferably the nucleic acid molecule comprises a cDNA encoding Fc_{ε}RI with the codon for Trp¹³⁰, Trp¹⁵⁶, Tyr¹⁶⁰ and/or Glu¹⁶¹ specifying alanine. Even more preferably the nucleic acid molecule is Trp¹³⁰-Ala, Asp¹⁵⁹-Ala, Tyr¹⁶⁰-Ala or Glu¹⁶¹-Ala as described in the Examples.

Alternatively preferably the nucleic acid molecule encodes an Fc receptor like peptide which has reduced ability to bind IgG or IgE. The phrase "reduced ability" has the same meaning as given earlier.

The amino acid alterations specified by the codons will generally be amino acids with different chemical characteristics such as that described earlier.

More preferably where the nucleic acid molecule encodes an Fc receptor-like molecule with a reduced ability to bind IgG the molecule comprises codons which result in one or more altered amino acids in the A/B, C/C' and/or B/F loops in the first domain and/or the B/C, C'/B and/or F/G loops in the second domain and/or the G/A strand.

Still more preferably the codons result in altered amino acids at positions 10-21, 42-48, 59-62, 113, 114, 115, 116, 129, 131 155 and/or 156.

Even more preferably the codons for the first domain of Fc_{γ}RII are removed or replaced by those for a receptor for another immunoglobulin.

Alternatively even more preferably the codons for Lys¹¹³, Pro¹¹⁴, Leu¹¹⁵, Val¹¹⁶, Phe¹²⁹, Arg/His¹³¹ and Ile¹⁵⁵ and/or Gly¹⁵⁶ are replaced by the codon for alanine in the Fc_{γ}RII. Still more preferably the nucleic acid molecule comprises the constructs D1eD2_{γ}, Lys¹¹³-Ala, Pro¹¹⁴-Ala, Leu¹¹⁵-Ala, Val¹¹⁶-Ala, Phe¹²⁹-Ala and/or Arg/His¹³¹-Ala described in the Examples.

Alternatively more preferably where the nucleic acid molecule encodes an Fc receptor-like molecule with a reduced ability to bind IgE the molecule comprises codons which result in one or more altered amino acids in the A/B, C/C' and/or E/F loops in the first domain or the F/G, C'/E and/or B/C loops in the second domain or the G/A strand.

Still more preferably the codons result in altered amino acids at positions 10-21, 42-48, 59-62, 129, 131, 132, 155, 158, 159.

Even more preferably the codons for the first domains of Fc_{ε}RI are removed or replaced by those for a receptor for another immunoglobulin.

Alternatively even more preferably the codons for Tyr¹²⁹, Tyr¹³¹, Glu¹³², Val¹⁵⁵, Leu¹⁵⁸ and/or Asp¹⁵⁹ is/are replaced by the codon for alanine in Fc_{ε}RI. Still more preferably the nucleic acid molecule of the invention comprises the constructs γ109-116ε, γ130-135ε, Tyr¹³¹-Ala, Glu¹³²-Ala, Val¹⁵⁵-Ala, Leu¹⁵⁸-Ala and Asp¹⁵⁹-Ala as described in the Examples.

In another preferred embodiment the invention relates to a nucleic acid molecule encoding a polypeptide with Fc binding ability wherein the polypeptide is altered compared to native Fc receptor such that the size of the polypeptide is larger than said native Fc receptor.

Preferably the nucleic acid molecule encodes a polypeptide that is soluble.

Preferably the nucleic acid comprises a component encoding naive Fc receptor, the extracellular region thereof or the Fc receptor-like molecule described earlier and a component encoding an amino acid sequence that results in the protein being larger than about 67 kD. Preferably the protein encoded is in the range of 67 kD to 1000 kD.

Preferably the non-Fc binding component encoded is a peptide well tolerated by an animal such as HSA, other Fc receptors, Igs from any species, cytokines and complement regulating molecules such as those discussed earlier. Even more preferably the nucleic acid has the same sequence as BSA : FcγRII or is substantially similar thereto.

In a further preferred embodiment the invention relates to a nucleic acid molecule encoding a polypeptide with Fc binding ability comprising a component capable of detection.

The term "component capable of detection" has the same meaning as given earlier.

Preferably the nucleic acid molecule encodes the polypeptide described in the first or second embodiments of the invention and preferably the component capable of detection is encoded by the appropriate nucleotide sequence such as a nucleotide sequence encoding an immunoglobulin, HRP, Alk-Phos or other detectable component.

The invention also extends to the nucleic acid molecules used as primers to produce the nucleic acid molecule of the invention. The primers described in the Examples are particularly preferred.

The invention further extends to a method of making the nucleic acid molecule of the invention comprising producing a nucleic acid encoding a polypeptide with Fc binding ability the structure of which is altered compared to a native Fc receptor by addition, deletion or substitution of one or more amino acids such that said alteration results in improved characteristics compared to said native receptor.

The term "producing a nucleic acid molecule" encompasses direct mutagenesis of a native Fc receptor gene by chemical means, SOE-PCR, *de novo* synthesis or addition of a nucleic acid such that a fusion protein is encoded. The different methods of effecting mutations will be well known to those skilled in the art.

The invention also extends to vectors comprising the nucleic acid molecules encoding the polypeptide with Fc binding ability described above and host cells expressing the nucleic acid molecules. Suitable vectors and host cells will be well known to those skilled in the art. In a preferred from the invention relates to the cDNA constructs and host cells containing them described in the Examples.

The invention also relates to a method of producing the polypeptides of the invention by recombinant means. The method comprises causing the nucleic acid molecule of the invention to be expressed and isolating or purifying the polypeptide to at least some degree. Generally the nucleic acid molecule will be present on a suitable vector or integrated into a host genome. Suitable hosts, vectors and purification methods will be known to those skilled in the art. However for the purposes of illustration only, some discussion of hosts and vectors is given below.

Suitable prokaryotic hosts include but not are limited to *Escherichia, Streptomyces, Bacillus* and the like. Suitable eukaryotic host include but are not limited to yeast, such as *Pichia* and *Saccharomyces* and animal cells in culture such as VERO, HeLa, mouse C127, Chinese hamster ovary (CHO), WI-38, BHK, COS, MDCK, NS1, J558 and insect cell lines. Such recombinant techniques have now become well known and are described in Methods of Enzymology, (Academic Press) Volumes 65 and 69 (1979), 100 and 101 (1983), and the references cited therein. An extensive technical discussion embodying most commonly used recombinant DNA methodologies can be found in Maniatis *et al*., Molecular Cloning, Cold Spring Harbor Laboratory (1982) or Current protocols in Molecular Biology, Greene Publishing (1988, 1991).

Once the nucleic acid encoding the polypeptide has been produced, the DNA may be introduced into an expression vector and that construction used to transform an appropriate host cell. An expression vector is characterised as having expression control sequences such that when a DNA sequence of interest is operably linked thereto, the vector is capable of directing the production of the product encoded by the DNA sequence of interest in a host cell containing the vector.

After the recombinant product is produced it is desirable to recover the product. If the product is exported by the cell producing it, the product can be recovered directly from the cell culture medium. If the product is retained intracellularly, the cells must be physically disrupted by mechanical, chemical or biological means in order to obtain the intracellular product.

With a protein product, it is desirable that the purification protocol provides a protein product that is essentially free of other proteins, and eliminates or reduces to acceptable levels other host cell contaminants, such as DNA, RNA, potential pyrogens and the like. Thus it may be desirable to use a commercially available system such as FLAG™ peptide system to allow easy purification. Alternatively the inherent Ig binding property of the polypeptide may be used to affinity purify it or anti-Fc receptor antibodies may be used.

As mentioned above, a variety of host cells may be used for the production of the polypeptide of the invention. The choice of a particular host cell is well within the knowledge of the ordinary skilled person taking into account, *inter alia*, the nature of the receptor, its rate of synthesis, its rate of decay and the characteristics of the recombinant vector directing the expression of the receptor. The choice of the host cell expression system dictates to a large extent the nature of the cell culture procedures to be employed. The selection of a particular mode of production be it batch or continuous, spinner or air lift, liquid or immobilised can be made once the expression system has been selected. Accordingly, fluidised bed bioreactors, hollow fibre bioreactors, roller bottle cultures, or stirred tank bioreactors, with or without cell microcarrier may variously be employed. The criteria for such selection are appreciated in the cell culture art.

In another embodiment the invention relates to a method of determining the presence of and/or amount of immunoglobulin in a sample said method comprising contacting said sample with the polypeptide of the present invention, or an Fc receptor or a part thereof, for a time and under conditions sufficient to allow the polypeptide or Fc receptor or part thereof and any immunoglobulin present in said sample to bind and detecting the presence of and/or determining the amount of said bound polypeptide-immunoglobulin, Fc receptor-immunoglobulin or part Fc receptor-immunoglobin.

The sample may be from any source where it is desired to determine the presence of immunoglobulin. Samples from body fluids and secretions such as blood, saliva, sweat, semen, vaginal secretions may be used. Solid tissue samples such as biopsy specimens from kidneys, etc, are also contemplated.

The term "an Fc receptor" refers to any native or non-native Fc receptor or a portion thereof whether derived from natural sources or by recombinant means. Preferably the Fc receptor is at least partly purified.

Detection of the bound polypeptide or Fc receptor can be determined by any convenient means. Preferably presence of immunoglobulin is detected by a reporter molecule. Alternatively the bound antibody-receptor may be detected by an anti-polypeptide labelled with a label, reporter molecule anti-Ig or other detectable signal. Determination of the amount of immunoglobulin may be made by comparison to a standard curve obtained using known quantities of Ig under identical conditions.

The polypeptide of the present invention or Fc receptor may be soluble or bound to a solid support such as nitrocellulose, paper, matrices or resins which will be known to those skilled in the art.

Another embodiment of the invention relates to a kit for detecting immunoglobulin including immune complexes in a sample, said kit comprising in compartmentalised form a first compartment adapted to receive the polypeptide of the invention or an Fc receptor and at least one other compartment adapted to contain a detector means.

The phrase "detector means* refers to means for detecting immunoglobulin bound to Fc receptor-like molecule and includes the appropriate substrates, buffers, etc required for detection of the bound immunoglobulin-receptor.

In this connection the polypeptide of the invention in the form of an Fc receptor-like molecule with enhanced ability to bind immunoglobulin of the present invention provides a useful laboratory reagent. This is particularly so with an Fc receptor-like molecule specific for IgG because the Fc receptor-like molecule is capable of selectively binding immunoglobulin complex. Thus the Fc receptor-like molecule including one with enhanced Ig binding ability may be used in immunoprecipitation as a replacement for protein A, for example, which does not exhibit a selective binding ability.

In a related embodiment the present invention provides a method of detecting immune complex in a sample comprising contacting said sample with the polypeptide of the invention or an Fc receptor specific for IgG for a time and under conditions sufficient for any complex present in the sample and the polypeptide or Fc receptor to form a further complex and detecting said further complex.

The above method utilises the ability of the polypeptide of the invention and the Fc receptor for IgG to bind complexes as they do not recognise monomeric IgG. Further the use of the Fc receptor-like molecule with enhanced activity provides a more sensitive assay as it will detect lower levels of complex in the sample and also be selective for the same.

The above method may be a useful tool in diagnosis of diseases where immune complexes are implicated such as, for example, glomerulonephritis, lupus, arthritis, heparin induced thrombocytopoenia thrombosis syndrome (HITTS), Gullien-Baré syndrome or idiopathic thrombocytopoenia pupuera (ITP). In a further embodiment relates to a method of removing immunoglobulin from a sample comprising contacting said sample with the polypeptide of the invention or an Fc receptor for a time and under conditions suitable for any immunoglobulin in the sample to form a complex with said polypeptide or Fc receptor and separating said complex from the remainder of the sample.

The sample used in the method may be any sample such as that described above or may be a sample continuously taken from a patient such as blood or plasma which is withdrawn, treated by the method and then returned to the patient as part of a closed system.

Preferably the polypeptide or Fc receptor used is specific for IgG and/or IgA. It has been noticed by the present inventors that FcγRII described herein is able to bind IgA. Thus, use of FcγRII would be particularly advantageous in the treatment of diseases involving immune complexes of IgG and/or IgA such as IgA nephropathies. Preferably the method is directed at removing immune complex existing in the sample and the polypeptide used is capable of binding immune complexes containing IgG or IgA.

Separation of the complex may be achieved by any convenient means such as by standard haemoperfusion or plasmaphoresis technologies but utilising a device containing the polypeptide of the present invention or a Fc receptor or a portion thereof bound to a solid support. Such a solid support includes silica, sephorose™, agarose, cellulose membranes, etc. such a device would preferably comprise a container enclosing the polypeptide or receptor or part thereof attached to a support, an inlet through which the sample can flow in and an outlet through which the sample can be returned to the patient.

In yet another embodiment the present invention relates to a method of removing immunoglobulin from a body fluid comprising taking body fluid from a patient, contacting the body fluid with the polypeptide of the invention or an Fc receptor, for a time and under conditions sufficient to allow the polypeptide to bind said immunoglobulin, removing said bound immunoglobulin from the body fluid and replacing said body fluid in the patient.

Preferably the method involves removal of immune complex. This may be used in the treatment of diseases where it is desirable to remove immune complex such as in lupus, IPT, HITTS, rheumatoid arthritis or after infection in a glomerulonephritis patient.

More preferably the method is used in plasmaphoresis in the treatment of immune complex diseases. Even more preferably the method utilises an Fc receptor-like molecule with enhanced ability to bind IgG.

Preferably the Fc receptor-like molecule is bound to a solid support such as a membrane when used in plasmaphoresis. Any suitable support could be used such as silica, agarose, sepharose™ or cellulose derivatives and will be known to those skilled in the art.

In another embodiment the present invention relates to a method of treatment of disease where the disease involves immune complexes or antigen-antibody interactions, said method comprising administering an effective amount of the polypeptide of the invention, an Fc receptor or an antagonist compound of the invention to a subject.

The subject may be a human or animal, preferably a mammal.

Preferably the polypeptide with an increased serum half life is used in the method of the invention. More preferably this polypeptide comprises the Fc receptor-like molecule with enhanced immunoglobulin binding ability is used in the method. In some diseases however an Fc receptor-like molecule with reduced immunoglobulin, or differential immunoglobulin binding ability may be indicated such as where it is desirable bind one form of immunoglobulin and not another. For example, an Fc receptor-like molecule with altered IgG binding ability such that it binds complexes and not monomers may be useful.

Preferably the polypeptides used in the method are soluble. More preferably they are administered in a pharmaceutical composition. The polypeptide of the invention optionally comprising the Fc receptor-like molecules with enhanced IgG binding ability may be used in the treatment of diseases where an excess of immunoglobulin is implicated as a causative agent of the inflammation or disease such as immune complex diseases, glomerulonephritis, lupus, rheumatoid arthritis, diseases involving inappropriate production of IgG after infection, heparin induced thrombocytopoenia thrombosis syndrome (HITTS) hyperacute graft rejection and idiopathic thrombocytopoenia pupuera (ITP).

In addition, the polypeptides of the present invention, Fc receptor-like molecules with enhanced IgG binding ability may be used in the treatment of any disease involving IgE, where IgE is one of the causative agents of disease. Such diseases include asthma, allergy and eczema.

Such a method comprises administering an effective amount of the polypeptide of the invention, or an Fc receptor, to a patient.

It is envisaged that the polypeptide of the invention, particularly a soluble one comprising IgE specific Fc receptor-like molecule with enhanced activity according to the invention will be particularly useful as a competitive inhibitor of IgE binding when administered to a subject. The polypeptide will function in two ways. First, it will absorb unbound IgE and second it will displace already bound IgE or prevent rebinding of IgE by virtue of its strong affinity for IgE. In this way the action of IgE in an asthma attack or allergic reaction such as in food allegy or bee sting may be reduced or alleviated.

Similar comments apply to a soluble IgG specific polypeptide of the invention. It is envisaged that particularly a soluble IgG specific Fc receptor-like molecule with enhanced activity according to the invention will be useful as a competitive inhibitor of IgG binding when administered to patients. First it will absorb to immune complexes aggregates or IgG which will prevent binding to cell surface F_{cγ}R, e.g. F_{cγ}RI, F_{cγ}RII, F_{cγ}RIII which will prevent or reduce activation of inflammation.

In this way immune complex induced inflammation, e.g. in rheumatoid arthritis, Good pastures syndrome, hyperacute graft rejection or lupus will be reduced or alleviated.

The invention will now be described with reference to the following non-limiting Examples.

### Example 1

### Materials and Methods

Chimeric Fc_{γ}RII/Fc_{ε}RI and mutant Fc_{γ}RII receptor cDNAs and expression constructs.

Chimeric Fc_{γ}RII/Fc_{ε}RIa chain or mutant Fc_{γ}RII cDNAs were constructed by Splice Overlap Extension (SOE) PCR (27) using the Fc_{γ}RIIa^{MR} cDNA (7) as template. SOB PCR was performed as follows: Two PCR reactions were used to amplify the Fc_{γ}RII-Fc_{ε}RI or Fc_{γ}RII fragments to be spliced together. The reactions were performed on 100ng of the Fc_{γ}RIIaNR cDNA in the presence of 500ng of each oligonucleotide primer, 1.25nM dNTPs, 50nM KCl, 10mM Tris-Cl pH 8.3 and 1.5nM MgCl₂ using 2.5 units of Taq polymerase (Amplitaq, Cetus) for 25 amplification cycles. A third PCR reaction was performed to splice the two fragments and amplify the spliced product. 100ng of each fragment (purified by size fractionation through an agarose gel) (28) was used with the appropriate oligonucleotide primers under the above PCR conditions.

The chimeric Fc_{γ}RII/Fc_{ε}RI a chain receptors were generated as follows. Chimera g, e109-116: oligonucleotide pairs (NR1 + CHM10) and (CHK09 + EG5) were used to produce two fragments which were spliced together using oligonucleotides NR1 and EG5. Chimera g, e130-135: oligonucleotide pairs (NR1 + PM12) and (PM11 + EG5) followed NR1 and EG5. The sequence of the oligonucleotide used and their positions of hybridisation with the Fc_{γ}RIIaNR cDNA are:

NR1, 5' - TACGAATTCCTATGGAGACCCAAATGTCTC-3', (nucleotide
position 10-30);

EG5, 5' - TTTGTCGACCACATGGCATAACG-3', (967-981);

CHMO9, 5' - CACATCCCAGTTCCTCCAACCGTGGCACCTCAGCATG-3', (419-
437 with nucleotides 442-462 of Fc_{ε}RI a chain);

CHM10, 5' - AGGAACTGGGATGTGTACAAGGTCACATTCTTCCAG-3',
(462-487 with 446-462 of Fc_{ε}RI a chain),

PM11, 5' - GTGGTTCTCATACCAGAATTTCTGGGATTTTCC-3', (473-490
with 492-506 of Fc_{ε}RI a chain);

PM12, 5' - CTGGTATGAGAACCACACCTTCTCCATCCCAC-3'' (516-531
with 491-506 of FcεRI a chain).

Sequences derived from Fc_{ε}RI a chain are underlined, Fc_{γ}RII not underlined, non-homologous sequences including restriction enzyme sites used in cloning of the PCR products are in bold type. Nucleotide positions refer to the previously published Fc_{γ}RIIa and Fc_{ε}RI a chain cDNA sequences (7, 13).

The Fc_{γ}RII Alanine point mutant cDNAs were generated using the following oligonucleotide combinations. Pro¹¹⁴-Ala, (GBC01 + EG5) and (GBC02 + MR1); Lys¹¹³-Ala: (GBCO3 + EG5) and (GBCO4 + NR1); Leu¹¹⁵-Ala, (BGCO5 + EG5) and (GBCO6 + NR1); Val¹¹⁶-Ala, (GBCO7 + EG5) and GBCO8 + NR1); Phe¹²⁹-Ala, (GCEO1 + EG5) and (GCEO2 + NR1); Ser¹³⁰-Ala, (GCEO3 + EG5) and GCEO4 + NR1); Arg/His¹³¹-Ala (GCEO5 + EG5) and GCEO6 + NR1); Leu¹³²-Ala, (GCEO7 + BG5) and GCEO8 + NR1); Asp¹³³-Ala, (GCEO9 + EG5) and (GCE10 + NR1); Pro¹³⁴-Ala, (GCE11 + EG5) and (GCE12 + NR1). Oligonucleotide NR1 and EG5 were used to splice together the two component fragments of each mutant to produce the point substituted cDNAs. The sequence of the oligonucleotides used and their positions of hybridisation with the Fc_{γ}RIIaNR cDNA are: NR1 and EG5 as described above; 
GBC01, 5'-GAAGGACAAGGCTCTGGTCAAG-3', (nucleotide position
443-464);
 GBCO2, 5'-CTTGACCAGAGCCTTGTCCTTC-3', (443-464); GBCO3, 5'-CTGGAAGGACGCTCCTCTGGTC-3', (440-461); GBCO4, 5'-GACCAGAGGAGCGTCCTTCCAG-3', (440-461); GBCO5, 5'-GGACAAGCCTGCTGTCAAGGTC-3', (446-467); GBCO6, 5'-GACCTTGACAGCAGGCTTGTCC-3', (446-467); GBC07, 5'-GACAAGCCTCTGGCTAAGGTCAC-3', (447-469); GBCO8, 5'-GTGACCTTAGCCAGAGGCTTGTC-3', (447-469); GCE01, 5'-CCCAGAAAGCTTCCCGTTTGG-3', (490-611); GCEO2, 5'-CCAAACGGGAAGCTTTCTGGG-3', (490-611); GCEO3, 5'-CAGAAATTCGCTCGTTTGGATC-3', (492-614); GCE04, 5'-GATCCAAACGAGCGAATTTCTG-3', (492-614); GCEO5, 5'-GAAATTCTCCGCTTTGGATCCC-3', (494-616); GCE06, 5'-GGGATCCAAAGCGGAGAATTTC-3', (494-616); GCEO7, 5'-ATTCTCCCGTGCTGATCCCACC-3', (497-619); GCEO8, 5'-GGTGGGATCAGCACGGGAGAAT-3', (497-619); GCEO9, 5'-CTCCCGTTTGGCTCCCACCTTC-3', (500-622); GCE10, 5'-GAAGGTGGGAGCCAAACGGGAG-3', (500-622); GCE11, 5'-CCGTTTGGATGCTACCTTCTCC-3', (503-625); GCE12, 5'-GGAGAAGGTAGCATCCAAACGG-3', (503-625). The Ala codon or its complement are shown in bold type.

Chimeric and mutant receptor cDNA expression constructs were produced by subcloning the cDNAs into the eukaryotic expression vector pKC3 (29). Each cDNA was engineered in the PCR reactions to have an EcoRI site at their 5' end (the 5'-flanking oligonucleotide primer NR1 containing an EcoRI recognition site), and a SalI site at their 3' end (the 3'-flanking oligonucleotide primer EG5, containing a SalI recognition site), which enabled the cDNAs to be cloned into the EcoRI and SalI sites of pKC3. The nucleotide sequence integrities of the chimeric cDNAs were determined by dideoxynucleotide chain-termination sequencing (30) using SequenaseTM (United States Biochemical Corp., Cleveland, OH) as described (31).

Transfections-COS-7 cells (30-50% confluent per 5cm² Petri-dish) were transiently transfected with FcR cDNA expression constructs by the DEM-dextran method (32). Cells were incubated with a transfection mixture (1ml/5cm2 dish) consisting of 5-10mg/ml DNA, 0.4mg/ml DEAE-dextran (Pharmacia, Uppsala, Sweden) and 1mM chloroquine (Sigma, St Louis, Mo) in Dulbecco's Modified Eagles Medium (DME) (Flow Laboratories, Australia) containing 10% (v:v) Nuserum (Flow Laboratories, Australia), for 4hr. The transfection mixture was then removed, cells treated with 10% (v:v) dimethysulphoxide in Phosphate buffered saline (PBS, 7.6mM Na₂HPO₄/3.25mM NaH₂PO₄/145mN NaCl) pH7.4 for 2 min, washed and returned to fully supplemented culture medium for 48-72 hr before use in assays. COS-7 cells were maintained in DME supplemented with 10% (v:v) heat inactivated foetal calf serum, 100U/ml penicillin, 100mg/ml streptomycin, 2mM glutamine (Commonwealth Serum Laboratories, Australia) and 0.05mM-2-mercaptoethanol (2mE) (Kock Light Ltd., UK).

Monoclonal antibodies and Ig reagents - The anti-Fc_{γ}RII mAb 8.2 was produced in this laboratory (19). The mouse IgE anti-TNP mAb (TIB142) was produced from a hybridoma cell line obtained from the American Type Culture Collection (Rockville, Ma); the mouse IgG1 anti-TNP mAb (A3) was produced from a hybridoma cell line which was a gift of Dr A Lopez (33). Human IgG1 myeloma protein was purified from the serum of a myeloma patient as described (34). Human IgG1 oligomers were prepared by chemical crosslinking using S-acetylmercaptosuccinic anhydride (SAMSA) (Sigma, St Louis, Mo) and N-Succinimidly 3-(2-pyridyldithio) propionate (SPDP) (Pierce Chemical Company, Rockford, IL) as follows: hIgG1 myeloma protein (5mg at 10mg/ml) in phosphate buffer (0.01M sodium phosphate pH 7.5/0.15M NaCl) was treated with a 5-fold molar excess of SPDP in dioxine, for 30 min. Excess reagents were removed by dialysis into PBS pH 7.0 / 2mM EDTA. The SAMSA modified hIgG1 was treated with 200ml hydroxylamine (1mM in PBS pH 7.0) for 30 min, then mixed with SPDP modified hIgG1 (1:1 molar ratio) and incubated for a further hr. The reaction was terminated with N-ethylmalemide (Sigma, St Louis, Mo) added to a final concentration of 6.6mM (35). All reactions were performed at room temperature. Dimeric hIgG1 was purified from monomeric and oligomeric hIgG1 by size fractionation chromatography on Sephacryl S-300 HR (Pharmacia LKB Biotechnology).

Erythrocyte-Antibody rosetting - COS-7 cell monolayers transfected with FcR expression constructs were incubated with EA complexes, prepared by coating sheep-red blood cells (SRBC) with trinitrobenzene sulphonate (TNBS) (Fluka Chemika, Switzerland) and then sensitising these cells with mouse IgG1 or IgE anti-TNBS mAb (36). Two ml of 2% EAs (v:v) were added per 5cm² dish of transfected cells and incubated for 5 minutes at 37°C. Plates were then centrifuged at 500g for 3 min and placed on ice for 30 min. Unbound EA were removed by washing with L-15 medium modified with glutamine (Flow Laboratories, Australia) and containing 0.5% Bovine serum albumin (BSA).

Direct binding of dimeric-hIgG1 or dimeric-mIgG1 - COS-7 cells transfected with FcR expression constructs were harvested, washed in PBS/0.5% BSA and resuspended at 107/ml in L-15 medium/0.5% BSA. 50ml of cells were incubated with 50ml serial dilutions of ¹²⁵I-dimeric-hIgG1 for 120 min at 4°C. ¹²⁵I-dimeric-Ig was prepared by the chloramine-T method as described (37) and shown to compete equally with unlabelled dimerio-Ig in binding to Fc receptor expressing COS-7 cells. Cell bound ¹²⁵I-dimeric-IgG1 was determined following centrifugation of cells through a 3:2 (v:v) mixture of dibutylphthalate and dioctylphthalate oils (Fluke Chemika, Switzerland) and cell bound ¹²⁵I-dimer determined. Non-specific dimer binding was determined by assaying on mock transfected cells and subtracted from total binding to give specific dimeric-IgG1 bound. Levels of cell surface Fc_{γ}RII expression were determined using the anti-Fc_{γ}RII mAb 8.2, shown to bind distantly to the binding site (19), and used to correct for variable cell surface receptor expression between the mutant Fc_{γ}RII COS-7 cell transfectants. The binding of 8.2 was determined in a direct binding assay as described for the human IgG1-dimer binding assays.

### RESULTS

Chimeric receptors identify multiple regions of Fc_{γ}RII involved in IgG binding.

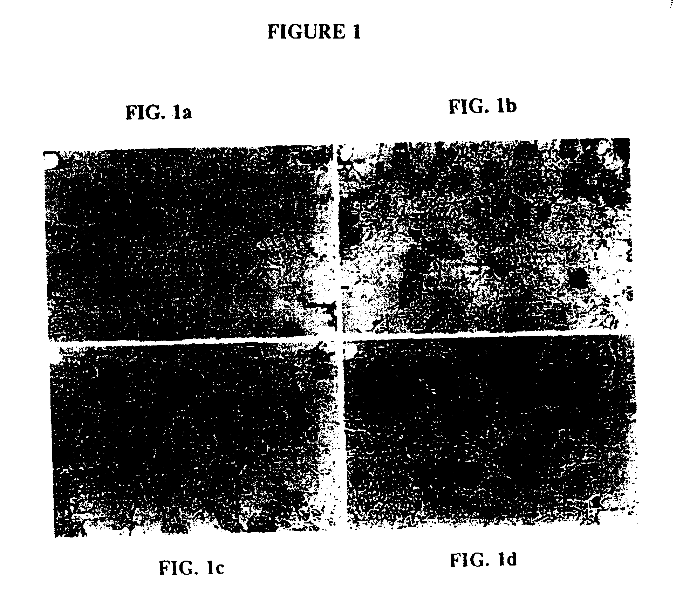

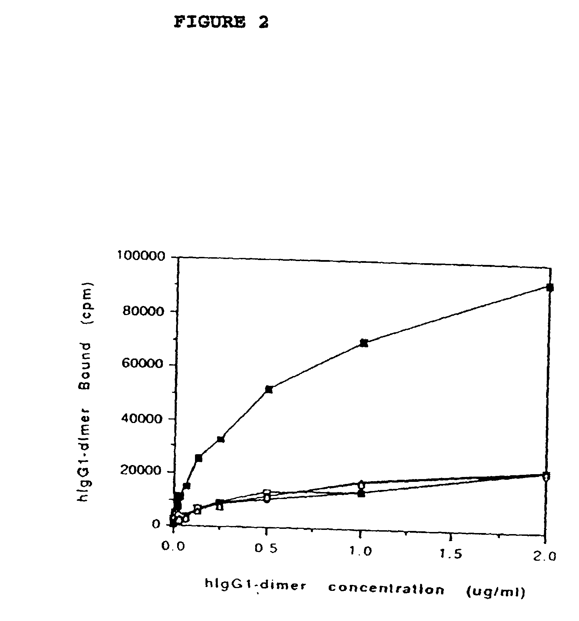

In order to determine the roles of domain 1 and the B-C or C'-E loop regions of domain 2 in the binding of IgG by human Fc_{γ}RII, chimeric receptors were generated whereby each of these regions in Fc_{γ}RII were replaced with the equivalent regions of the human Fc_{ε}RI a chain. Chimeric receptor cDHAs were constructed by SOE PCR, subcloned into the eukaryotic expression vector pKC3 and transiently transfected into COS-7 cells. The IgG binding capacities of the chimeric receptors were determined by both EA rosetting and the direct binding of dimeric hIgG1. The substitution of Fc_{γ}RII domain 1 with that of the Fc_{ε}RI a chain produced a receptor (designated D1_{ε}D2_{γ}) which as expected retained the capacity to bind the highly sensitised IgG-EA complexes (Fig. 1a), however in contrast to the wild-type receptor did not bind dimeric-hIgG1 (Fig. 2). Similarly, the replacement of the region of the Fc_{ε}RI α chain comprising residues Ser¹⁰⁹-Val¹¹⁶ (B-C loop) or Ser¹³⁰-Thr¹³⁵ (C'-E loop) of Fc_{γ}RII domain 2 with the equivalent regions of the Fc_{ε}RI a chain (producing chimeras γ109-116ε and γ130-135ε respectively), also resulted in the loss of hIgG1-dimer binding (Fig. 2), yet these receptors retained the ability to bind IgG-EA complexes (Fig. 1b,c). COS-7 cells transfected with an expressible form of the Fc_{ε}RI α chain (17) did not bind either hIgG1 dimers of IgG-EA (Fig. 1d, Fig. 2). Thus the ability of chimeric Fc_{γ}RII containing domain 1 or B-C, C'-B domain 2 substitutions to bind the highly substituted EA complexes but not dimeric-hIgG1, suggests that these receptors bind IgG with a lower affinity than wild-type Fc_{γ}RII. These findings demonstrate that although the domain 1 and domain 2 B-C, C'-B regions do not seem to directly bind IgG, they do appear to make a contribution to the binding of IgG by Fc_{γ}RII.

### Fine structure analysis of the B-C and C'-E loops of Fc_{γ}RII domain 2.

The contribution of the B-C and C'-E loop regions of Fc_{γ}RII to the binding of IgG was determined using a point mutagenesis strategy where individual residues in both the B-C (residues Lys¹¹³-Val¹¹⁶) and C'-E (residues Phe¹²⁹-Pro¹³⁴) loops were replaced with alanine. cDNAs encoding the mutant receptors were also generated using SOB PCR and subcloned into the eukaryotic expression vector pKC3. The resultant expression constructs were transiently transfected into COS-7 cells and the Ig binding capacity of the mutant receptors determined by assessing the binding of dimeric hIgG1. The levels of cell membrane expression of the mutants on the COS-7 cell transfactants were determined using the anti-Fc_{γ}RII mAb 8.2 (shown to detect an epitope distant to the binding site) and were comparable to the of the wild-type receptor (see legend Fig. 3). The relative capacity of the mutant receptors to bind hIgG1 were determined using the direct binding assay following correction for variation in cell surface expression levels, and expressed as percentage of wild-type Fc_{γ}RII binding.

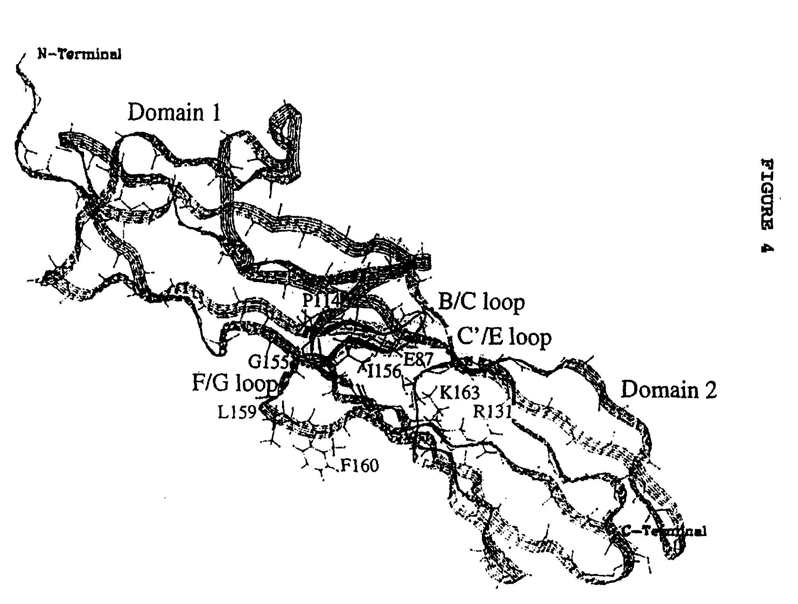

The replacement of the B-C loop residues (Lys¹¹³, Val¹¹⁴, Leu¹¹⁵, Pro¹¹⁶) in turn with Ala, in each case resulted in diminished hIgG1-dimer binding (Fig. 3). The most dramatic effect was seen on substitution of Lys¹¹³ and Leu¹¹⁵, which exhibited only 15.9 + 3.4 (mean + SD) and 20.6 + 4.0 percent binding compared to wild-type Fc_{γ}RII. The replacement of Val¹¹⁴ or Pro¹¹⁵ with Ala had a lesser effect, these receptors displaying 53.5 + 13.5 and 73.5 + 7.9 percent wild-type binding respectively. These results suggest that each of these residues in the B-C loop contribute to the binding of IgG by Fc_{γ}RII, whether as direct contact residues or indirectly by maintaining the correct conformation of the binding site. Alanine replacement of residues 129 to 134 of the C'-E loop (Phe¹²⁹, Ser¹³⁰, Arg/His¹³¹, Leu¹³², Asp¹³³, Pro¹³⁴) also suggests this region plays a role in the binding of IgG by Fc_{γ}RII. Substitution of Phe¹²⁹ and Arg/His¹³¹ decreased hIgG1-dimer binding by over 90% and 80% respectively to 8.2+ 4.4 and 21.9 + 3.9 compared to that of wild-type Fc_{γ}RII (Fig. 3). In contrast, replacement of residues Asp¹³³ and Pro¹³⁴ increased binding to 113.5 + 8.8 and 133.5 + 0.2 percent of the wild-type receptor. The substitution of Ser¹³⁰ or Leu¹³² had no significant effect on the binding of hIgG1, as these mutants exhibited comparable binding to wild-type Fc_{γ}RII (Fig. 3). These findings suggest Phe¹²⁹ and Arg/His¹³¹ may play an important role in the binding of hIgG1, and the observation that the substitution of Asp¹³³ and Pro¹³⁴ increase binding also suggest an important role for these residues, which appears different from Phe¹²⁹ and Arg/His¹³¹. Again, a distinction between a possible direct binding role or contribution to structural integrity of the receptor cannot be made, however these findings clearly identify both the B-C and C'-E loops as playing a role in the binding of IgG by Fc_{γ}RII. The positions of the residues proposed to have binding roles on the putative domain 2 model suggests that it is the region of the B-C-C'-E-F-G face forming the interface with domain 1 that is involved in the contact of Fc_{γ}RII with IgG (Fig. 4).

### DISCUSSION

The findings presented herein provide evidence to suggest that the interaction of IgG with hFc_{γ}RII involves multiple regions of the receptor. Of the entire extracellular region, only the 154-161 segment was demonstrated to directly bind IgG, since insertion of only this region into the corresponding region of the human Fc_{ε}RI a chain, imparted IgG binding function to Fc_{ε}RI. Moreover, replacement of this region in Fc_{γ}RII with that of Fc_{ε}RIa resulted in loss of IgG binding, implying that residues Asn¹⁵⁴ to Ser¹⁶¹ of Fc_{γ}RII comprises the key IgG1 interactive site of hFc_{γ}RII. However, the generation of further chimeric hFc_{γ}RII/Fc_{ε}RIa receptors as described in this application has suggested that two additional regions of hFc_{γ}RII domain 2, although not directly capable of binding IgG, also influence the binding of IgG by hFc_{γ}RII. The replacement of the regions encompassing Ser¹⁰⁹ to Val¹¹⁶ (B-C loop) and Phe¹²⁹ to Pro¹³⁴ (C'-E loop) of hFc_{γ}RII with the equivalent regions of the Fc_{ε}RI a chain, produced receptors which despite containing the putative binding site (λsn¹⁵⁴ to Ser¹⁶¹) and retaining the ability to bind IgG-EA, lost the capacity to bind dimeric hIgG1. Indeed, site-directed mutagenesis performed on each individual residue of the 109-116 and 129-134 regions identified a number of residues which appear to play crucial roles in hIgG1 binding by Fc_{γ}RII. The replacement of Lys¹¹³, Pro¹¹⁴, Leu¹¹⁵ and Val¹¹⁶ of the B-C loop, and Phe¹²⁹ and Arg/His¹³¹ of the C'-B loop with alanine, all resulted in diminished hIgG1 binding. Therefore, these findings provide strong evidence to suggest that the B-C and C'-E loops of hFc_{γ}RII also contribute to the binding of IgG.

The findings described herein suggest the nature of the residue at 131 plays a role in the binding of hIgG1, as replacement with alanine results in a marked reduction in binding of this isotype to hFc_{γ}RII. Thus, although the F-G loop of hFc_{γ}RII is clearly the major region involved in the direct interaction with IgG, as demonstrated in that only this region has been definitively shown to directly bind IgG (20), residue 131 also appears to play a binding role.

The molecular model of the entire F_{cγ}RII shows that the regions involved in Ig binding are located on the same face of domain 2 and at the interface between domains 1 and 2. Furthermore, this also indicates that the A/B and E/F loops of domain 1 as well as the strand connecting domains 1 and 2 (G/A strand) are located in the same region (interdomain interface) and contribute to the binding area of the domain. This area forms a hydrophobic pocket and development of receptor antagonists would be targeted at this region.

Furthermore since F_{cγ}RII and F_{cε}RI as well as other F_{c}R are homologous then their overall structure and general principles of the location of the binding sites would be similar to that disclosed in this application.

The studies described herein demonstrate that domain 1 of hFc_{γ}RII, although does not appear to play a direct role in IgG binding, does play an important role in the affinity of IgG binding by hFc_{γ}RII. This is suggested as replacement of domain 1 of hFc_{γ}RII with domain 1 of hFc_{ε}eRI, reduced the capacity to bind IgG, as shown by the failure of this receptor to bind dimeric hIgG1. These data imply that the IgG binding role of domain 1 is likely to be an influence on receptor conformation, stabilizing the structure of domain 2 to enable efficient IgG binding by hFc_{γ}RII. This proposal is consistent with the localisation of the IgG binding site of hFc_{γ}RII to loop regions in domain 2 at the interface with domain 1. The binding site is therefore in close proximity to domain 1 and as such predicted to be influenced in conformation, presumably by the loop and strand region at the bottom of domain 1 i.e. G strand, and the A-B, E-F and C'-C loops.

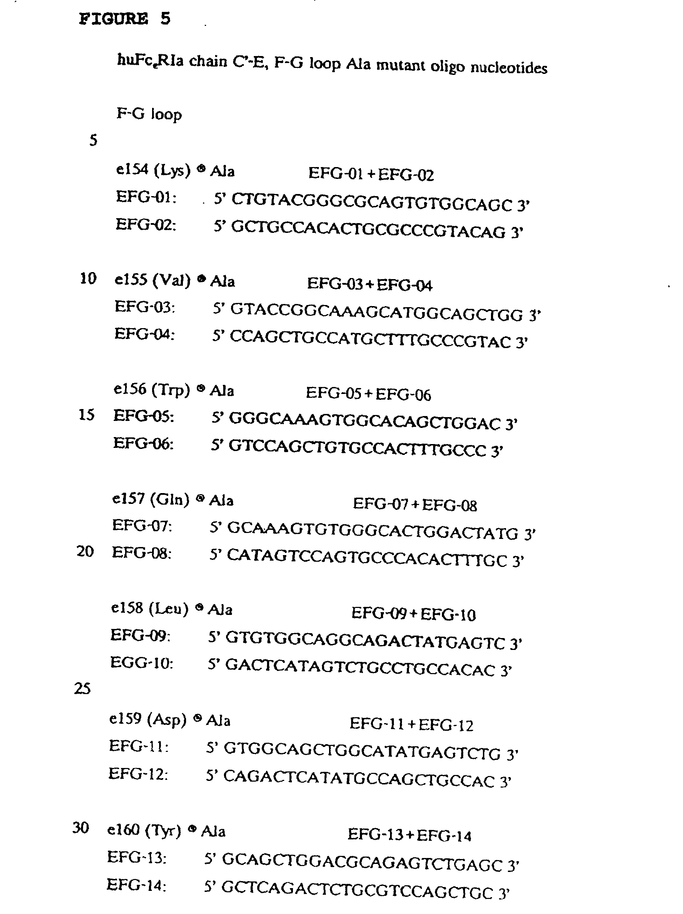

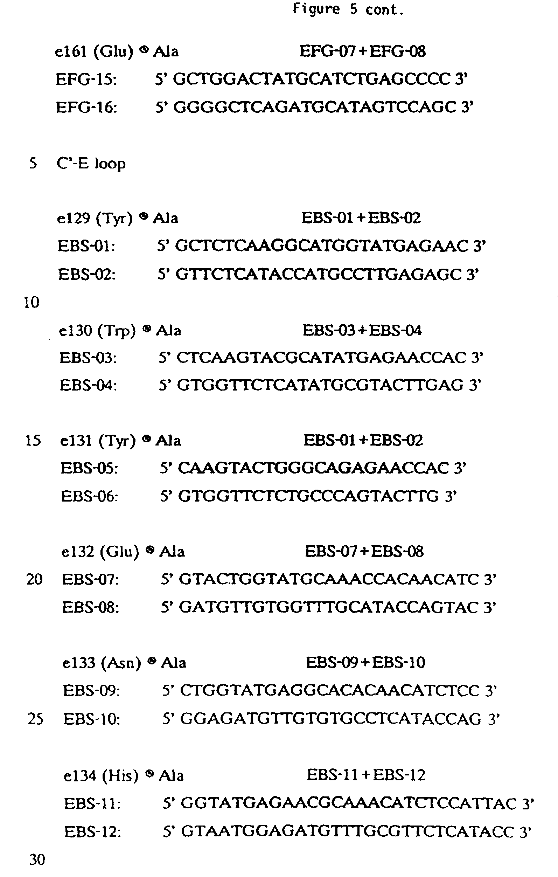

Further support for the involvement of the B-C and C'-E loops of hFc_{γ}RII domain 2 in the binding of IgG has been provided in the cloning and subsequent Ig binding studies of rat Fc_{γ}RIII (38), which is structurally and functionally homologous to Fc_{γ}RII. Two rat Fc_{γ}RIII isoforms, IIIA and IIIH, which have extensive amino acid differences in their second extracellular domains, have been shown to bind rat and mouse IgG subclasses differently. Both isoforms bind rtIgG1 rtXgG2b and mIgG1, however differ in that only the IIIH form binds rtIgG2b and mIgG2b. Significantly, the amino acid differences between rat Fc_{γ}RIIIA and IIIH isoforms are situated predominantly in the predicted B-C and C'-E loops of domain 2 (Fig. 5). However, it should be noted that the F-G loop regions of rat Fc_{γ}RIIIA and IIIH are almost totally conserved, which together with the observation that both forms bind rtIgG1 rtIgG2a and mIgG1, is consistent with the proposal that the F-G loop region is the major IgG interactive region, and that the B-C and C'-B loop regions provide supporting binding roles.

It is interesting to note that a number of parallels are also apparent in the molecular basis of the interaction of hFc_{γ}RII with IgG and that of hFc_{ε}RI with IgE. The Ig binding roles of the two extracellular domains of hFc_{ε}RI are similar to hFc_{γ}RII, with domain 2 responsible for the direct binding of IgE and domain 1 playing a supporting structural role (17,26). Furthermore, as described for hFc_{γ}RII, we have also identified multiple IgE binding regions in domain 2 of hFc_{ε}RI. Using chimeric hFc_{γ}RII/Fc_{ε}RI receptors we have demonstrated that domain 2 of hFc_{ε}RI contains at least 3 regions each capable of directly binding IgE, as the introduction of the Fc_{ε}RI regions encompassed by residues Trp⁸⁷ to Lys¹²⁸, Tyr¹²⁹ to Asp¹³⁵ and Lys¹⁵⁴ to Gln¹⁶¹ into the corresponding regions of hFc_{γ}RII was found to impart IgE binding to hFc_{γ}RII (17, 20). These data suggest at least 4 regions of hFc_{ε}RI domain 2 contribute to the binding of IgE, Ser⁹³ to Phe¹⁰⁴, Arg¹¹¹ to Glu¹²⁵, Tyr¹²⁹ to Asp¹³⁵ and Lys¹⁵⁴ to Ile¹⁶¹. Three of these regions correspond to the 3 regions identified herein as important in the binding of IgG by Fc_{γ}RII, Arg¹¹¹ to Glu¹²⁵, Tyr¹²⁹ to Asp¹³⁵ and Lys¹⁵⁴ to Ile¹⁶¹, which encompass the B-C, C'-E and F-G loops respectively. Thus, as described herein for hFc_{γ}RII, these findings implicate the B-C, C'-E and F-G loops juxtaposed in domain 2 at the domain 1 interface, as the crucial IgE interactive region of hFc_{ε}RI.

### EXAMPLE 2 Characterisation of Domain 2 of FcγRII MATERIALS AND METHODS

Mutants were constructed and constructs used to transfect COS cells as in Example 1.

### RESULTS AND DISCUSSION

Additional mutants of domain 2 of FcγRII were made. Three point mutations where a single amino acid residue was changed to alanine were constructed. The mutant cDNAs were used to transfect COS cells and tested for the ability of the proteins encoded by the mutants to bind human IgG1 dimers. Mutations at Asn¹²³ and Gly¹²⁴ has no effect on binding whereas mutation at Lys¹²⁵ was found to decrease binding (data not shown). The amino acid residues tested are found in the space between the C' and the C loops. The results indicate that the 123 and 124 positions do not contribute to binding site and that the 125 position is involved in binding. Construction of C' - C hFcγRII Ala point mutant CDNAs

Asn¹²³-Ala, CC-01 + EG5 and CC-02 + NR1

Gly¹²⁴-Ala, CC-03 + EG5 and CC-04 + NR1

Lys¹²⁵-Ala, CC-05 + EG5 and CC-06 + NR1

Oligonucleotide sequences and their positions of hybridization with the FcγRII a^{MR} cDNA are as follows:

CC-01, 5'-CATTCTTCCAGGCAGGAAAATCCCAG-3', (nucleotide
position 467-498);

CC-02 5'-CTGGATTTTCCTGCCTGGAAGAATG-3', (467-494)

CC-03 5′-CTTCCAGAATGCAAAATCCCAGAAATTC-3′, (473-500);

CC-04 5′-GAATTTCTGGGATTTTGCATTCTGGAAG-3′, (473-500);

CC-05 5′ -CCAGAATGGAGCATCCCAGAAATTC-3′, (476-500);

CC-06 5′ -GAATTTCTGGGATGCTCCATTCTGG-3′, (476-500).

### EXAMPLE 3 Comparison of the binding of human IgG1 and IgG2 to the Alanine mutants of FcγRII

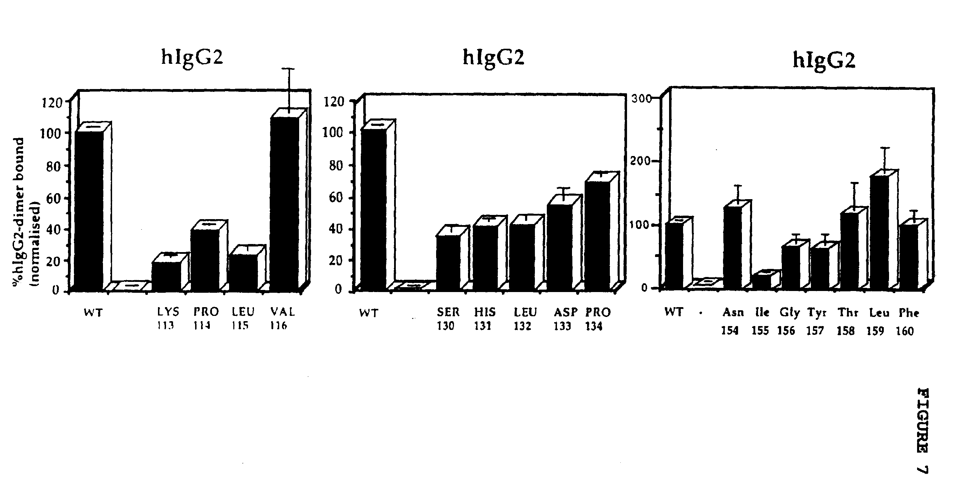

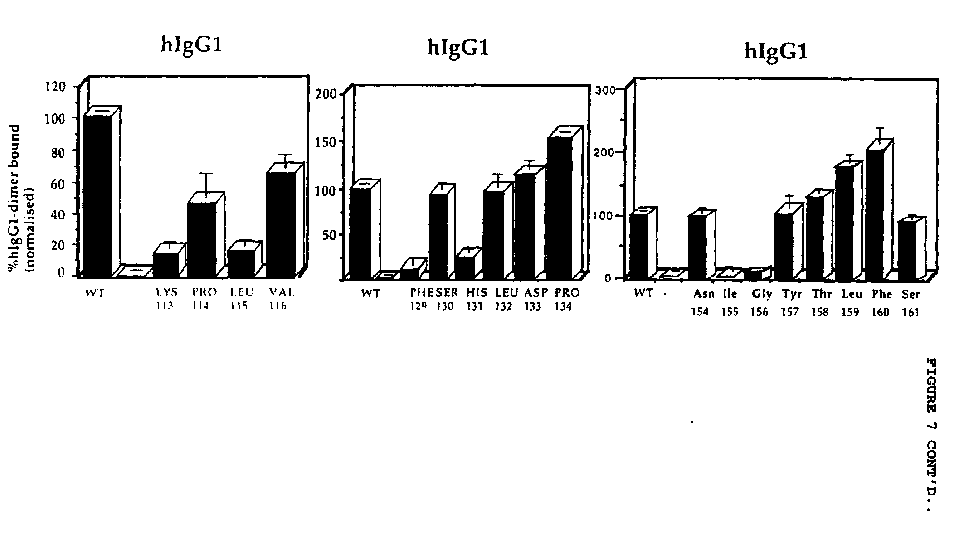

The binding of human IgG2 was also assessed and some similarities and differences in the nature of mutations that affect binding of IgG1 or IgG2 were observed (Fig.7).

Mutations of Lys¹¹³, Pro¹¹⁴, Leu¹¹⁵, Phe¹²⁹, His¹³¹, I¹⁵⁵, G¹⁵⁶ decrease IgG1 and IgG2 binding.

Mutation of Val¹¹⁶ decreases IgG1 binding only.

Mutation of Ser¹³⁰, Leu¹³², Asp¹³³, Pro¹³⁴, Tyr¹⁵⁷ reduces IgG2 binding only.

Mutation of Thr¹⁵⁸ and Leu¹⁵⁹ also enhances IgG1 and IgG2 binding.

### EXAMPLE 4 The IgE Binding Site of Fc,RI

Similar experiments to those described for the IgG receptor Fc_{γ}RII were performed on the IgE receptor, Fc_{ε}RI.

Three regions of the IgE receptor were the target of mutagenesis experiments. These regions defined by residues 112 to 116, 129 to 134 and 154 to 161 are located in the second domain of Fc_{ε}RI. The experiments where performed wherein individual amino acid residues were mutated to alanine and the effect on IgE binding measured. Mutation of the Fc_{ε}RI was performed by splice overlap extension on described for the Fc_{γ}RII using the oligonucleotides shown in Fig. 5.

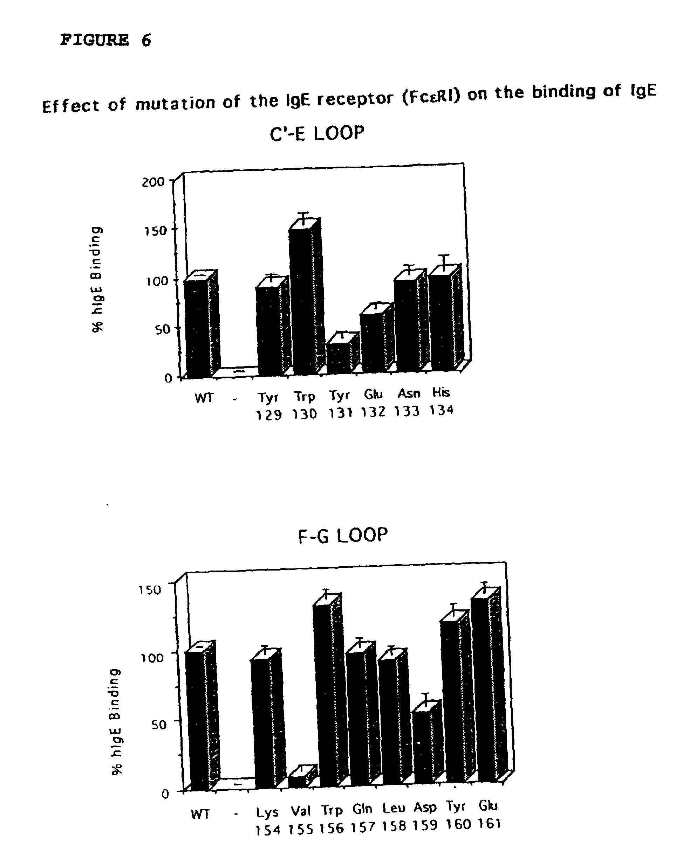

Mutation of Tyr¹³¹ or Glu¹³² profoundly decreased the capacity of Fc_{ε}RI to bind IgE (Fig. 6). By contrast mutation of Trp¹³⁰ resulted in enhancement or improvement of IgE binding by Fc_{ε}RI.

Mutation of the residues in the segment from (and including) residue 154-161 also showed that mutation of Val¹⁵⁵ completely ablated binding and mutation of Leu¹⁵⁸ or Asp¹⁵⁹ also decreased IgE binding. Furthermore mutation of Trp¹⁵⁶, Tyr¹⁶⁰ or Glu¹⁶¹ enhanced IgE binding to Fc_{ε}RI. Since domain 1 is also involved in Ig binding and since we have developed a molecular model of Fc_{γ}RII and since we know Fc_{γ}RII and Fc_{ε}RI are highly related proteins it is likely that similar regions of Fc_{ε}RI domain 1 to those of Fc_{γ}RII will be involved in binding i.e. in Fc_{ε}RI the, A/B loop residues Asn¹⁰ - Asn²¹, C/C' loop, residues Asn⁴² - Glu⁴⁷ and the B/F loop residues Lys⁵⁹ - Asp⁶².

On the basis of these studies it is clear that certain residues have a major role in Fc_{ε}R interaction and that manipulation of these residues would be useful in the production of useful pharmaceutical or diagnostic reagents. Thus mutation of Tyr¹³¹, Glu¹³², Val¹⁵⁵, Leu¹⁵⁸, Asp¹⁵⁹ all decrease IgE binding. Conversely mutation of Trp¹³⁰, Trp¹⁵⁶, Tyr¹⁶⁰ or Glu¹⁶¹ all improve Fc_{ε}RI function since these mutant receptors are able to bind IgE more effectively than the wild type receptor.

### EXAMPLE 5 Effect of Chimeric Receptors on IgE Binding MATERIALS AND METHODS

Chimeric receptors were made as described in Example 1. Chimeric receptors were produced which have Domain 2 of the FcεRII but have varying components in Domain 1. The terminology is as follows:
- εεγ :: denotes a receptor with Domains 1 & 2 from FcεRII and a transmembrane region from FcγRII. This was used as a control.
- γεγ :: denotes a receptor with Domain 1 from FcγRII, Domain 2 from FcεRII and a transmembrane region from FcγRII.
- G :: denotes γεγ which contains the G strand from FcεRI
- EF :: denotes γεγ which contains the E/F loop from FcεRI
- CC':: denotes γεγ which contains the CC' loop from FcεRI

The oligonucleotides used to create the above chimeric receptors were as follows :

| | |
|---|---|
| CC' | NR1 + LR3 |
| | LR4 + EG5 |
| EF | NR1 + EG32 |
| | EG33 + EG5 |
| G | NR1 + LR1 |
| | LR2 + EG5 |

NR1 and EG5 are as described in Example 1.

Antisense LR1 5' - GGTTCACTGAGGCTGGTCTGGC-3'

Sense LR2 5' - CAGCCTCAGTGAACCTGTGTACC-3'

Antisense LR3 5′ - CGTCTCTTCTGACAGGCTGCCATTGTGGAACCAC-3′

Sense LR4     5' - GTCAGAAGAGACGAATTCACCCAGCTACAGGTCC-3'

Antisense EG32 5' - AAATTTGGCATTCACAATATTCAAGCTGGGCTGCGTGTGG-3'

)    Sense EG33    5'-
AATATTGTGAATGCCAAATTTGAAGACAGCGGGGAGTACAC-3'

The efficiency of binding of the chimeric receptors to IgE was assayed using IgE coated erythrocytes on monolayers of COS cells transfected with cDNA encoding the constructs described directly above. Radiolabelled Fc portion of IgE was used in the study of the chimeric receptors.

### RESULTS & DISCUSSION

The results of the rosetting assay showed that the cells transfected with the CC' chimeric construct rosette less well (data not shown) than the other transfectants.

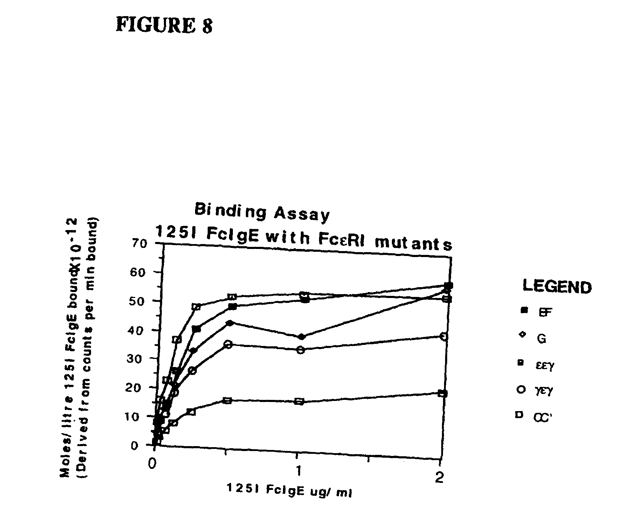

The chimeric receptors were subjected to a quantitative assay using radiolabelled Fc portion of IgE (see Figure 8). The chimeric receptor γεγ restores binding to the same level as that seen in the normal receptor (εεγ in this case). This implies that the BF and the G regions are important in binding in FcεRII.

### EXAMPLE 6 Production of a soluble polypeptide with Fc binding ability and production of the FCγRII Fusion Protein

Two examples of the genetically engineered polypeptide of the invention are recombinant soluble FcγRII (which consists only of the extracellular domains of FcγRII) and a fusion protein consisting of human serum albumin genetically fused to the extracellular domains of FcγRII.

The recombinant soluble Fc receptor (rec. sFCγRII) can be generated using standard mutagenesis techniques including splice overlap extension (SOE) as described earlier. The FcγRIIcDNA or genomic DNA or a combination thereof is mutated such that a translation termination codon (e.g. TAA,TGA OR TAG) is inserted into the DNA in a position that will terminate the translation of RNA derived from such mutant DNA to yield proteins containing the Ig binding extracellular region without the transmembrane anchoring segment.

Thus a molecule was generated by using SOB to introduce the codon, TAG, 3' of the residue 170. This mutated DNA was introduced into several expression systems and a polypeptide with Fc binding ability was obtained. The expression systems included CHO cells, fibroblasts, a yeast (*Pichia pastoris*) and bacculovirus. The molecular weight of the rec.sFcγRII varies according to the expression system, ie. 30 KD from CHO cells or 26 KD from *Pichia pastoris*. This is due to differences in glycosylation. Clearly many other expression systems could be used including bacteria, plants and mammals.

The second example of a polypeptide of the present invention is a fusion protein which is produced by fusing DNA encoding a polypeptide with Fc binding ability to DNA encoding a different protein to generate a new protein which retains Fc binding ability. Such new protein would include human serum albumin fused to FcγRII. This protein can be generated using SOB to fuse the DNA encoding HSA to FcγRII. This is done in such a way that the residues near the C terminus of HSA are fused to amino acid residues in the amino terminus of FcγRII. It is important to note that although in this example FcγRII encoding DNA is used in the fusion protein, it is also possible to use DNA encoding other proteins such as the DNA encoding the Fc receptor-like molecules described earlier.

### MATERIALS & METHODS

The HSA:FCγRII fusion protein was produced according to the following method. Oligonucleotides HT4 an HT7 were used to amplify the HSA DNA. HT4 contains the restriction site (Eco RI) for cloning and HT7 contains a sequence that overlaps with FcγRII. The sequences are as follows:

HT4 5′ ATCGATGAATTCATGAAGAAGTGGTGGGTAAC 3′

HT7 5′ GGGGGAGC/GCCTAAGGCAGCTTGAC 3′

The Eco RI restriction site is shown in bold in HT4 above. Just adjacent to this is the ATG which is the HSA start codon. A slash in the HT7 sequence shown above denotes the FcγRII-HSA junction.

Oligonucleotides HT8 and HT5 were used to amplify the required segment (extracellular domains) of FcγRII. HT8 contains a sequence that overlaps with the HSA sequence (and also oligonucleotide HT7). HT5 also contains a translation termination codon as well as a restriction site (Eco RI) for cloning purposes. The sequences of the oligonucleotides are as follows :

HT8 5'S CCTTAGGC/GCTCCCCCAAAGGCTG 3'

HT5 5′ CCCCATCATGAATTCCTATTGGACAGTGATG 3′

The slash in the HT8 sequence shown above denotes the junction between HSA and FcγRII encoding DNA. The Eco RI site in HT5 is denoted by bold type. The termination codon, CTA is adjacent to the Eco RI site. Note that HT7 and HT5 are antisense sequences.

The constructs made were used to transform *Pichia pastoris* cells. Western blots of the proteins expressed were conducted.

### RESULTS & DISCUSSION

A Western blot was performed using a polyclonal anti-FcγRII antibody. The supernatants from the transformed yeasts were tested with the antibody and the results demonstrated that only the supernatants from HSA:FcγRII transfected cells and the cell transfected with a construct encoding soluble FcγRII reacted with the antibody. The controls did not react (results not shown).

The Western blotting detected a protein of approximately 100KD which is of the expected molecular weight being 67KD from HSA plus 30kD from FcγRII extracellular domains. A recombinant FcγRII of 30kD was also detected by the antibody.

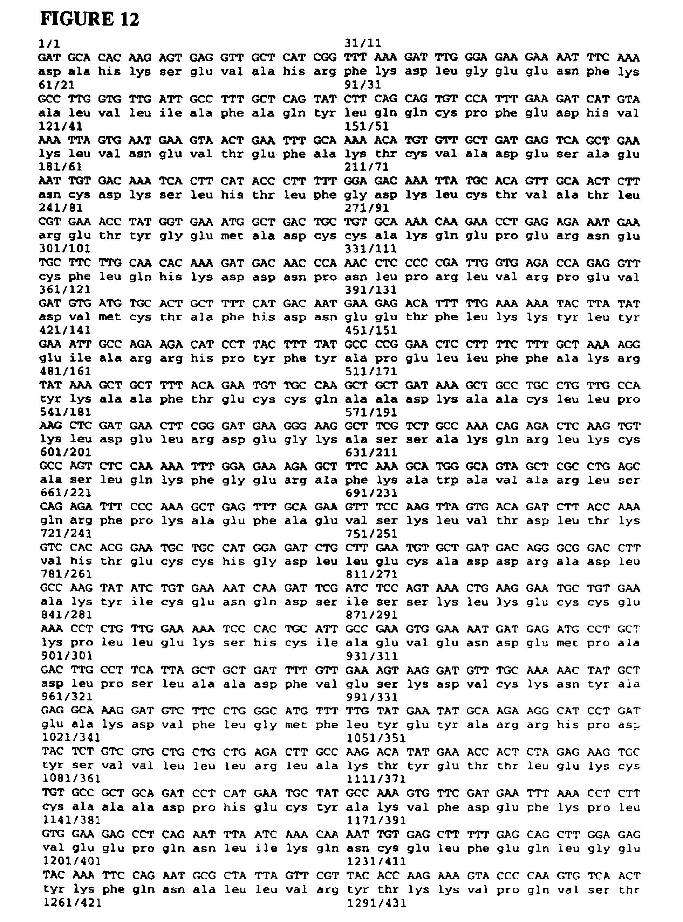

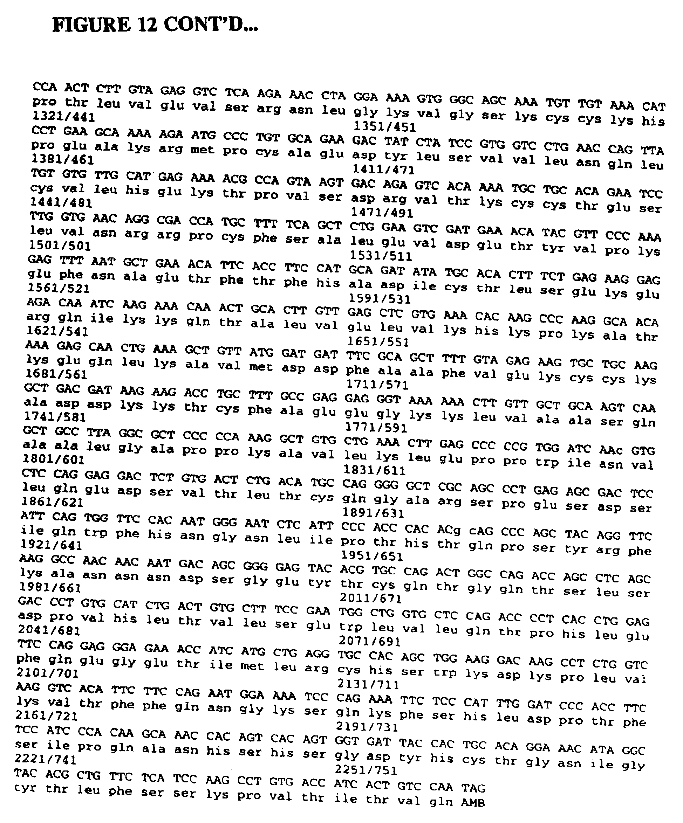

The 100KD protein produced bound to immunoglobulin coated beads but not to Fab'2 coated beads indicating that it has specificity for the Fc portion of IgG. In addition sequencing of the SOB produce confirmed the predicted sequence of the fusion protein as shown in Fig. 12.

It is clear that FcγR extracellular domains can be attached to additional molecules and still retain Fc receptor activity. Since there are a number of Fc receptors closely related to the FcγRII, especially in their Ig binding extracellular regions, it is clear that modifications of the type described for FcγRII would also be possible for these other Fc receptors such as FcγRI, FcεRI, FcγRIII and FcαRI. This appears to be particularly the case when the receptors mentioned immediately above have essentially the same number of amino acids in the extracellular regions. In addition the FcεRI, FcγRII, FcαR and FcεRIII all have extracellular regions that are organised into two di-sulphide bonded domains that are members of the immunoglobulin super family. Furthermore, the ligands for FcγRII, FcγRIII, FcεRI and FcαR are all homologous.

It is also clear that the non-Fc binding portion of the fusion protein may be attached to the other species receptors discussed above. The "foreign" component of the fusion protein may be an immunoglobulin provided that the immunogolobulin would be a type unable to bind the Fc binding portion of the fusion peptide. For example, the extracellular portion of FcγRII could be attached to IgM or the extracellular portion of FcεRI could be attached to IgG. Other foreign protein components could include ovalbumin, other Fc receptors, compliment, other recombinant derived proteins including CD46, CD59, DAF, CRI, CR3 and proteins especially those involved in regulating inflammation including cytokines and complement regulating proteins. The receptor could also be attached to other high molecular weight entities.

Although the above experiments contemplate production of polypeptides according to the invention via recombinant means it is also possible that polypeptides according to the invention could be generated by other non-recombinant means. This could be achieved by chemical means such as attaching the Fc binding portion of the protein to other molecules such as dextrans, lipids, and carbohydrates with the proviso that the molecules produced retain Fc binding ability.

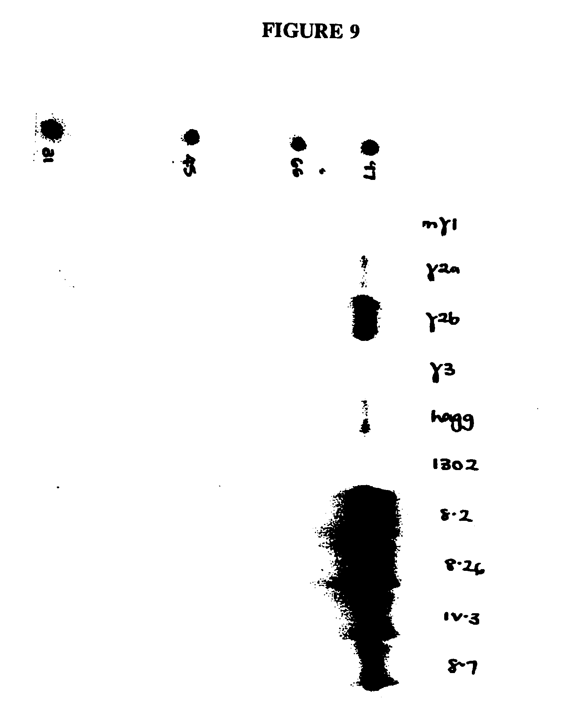

Figure 9 shows that HSA-FcγRII is specific for immunoglobulin, and that the fusion protein is correctly folded since the epitopes detected by the monoclonal antireceptor antibodies are intact. It also demonstrates that HSA-FcγRII binds mouse IgG1 (mγ1), IgG2b (γ2b) and human IgG (HAGG) but it does not bind IgG lacking an Fc portion (1302).

### EXAMPLE 7 HSA:FcγRII administered to an animal.

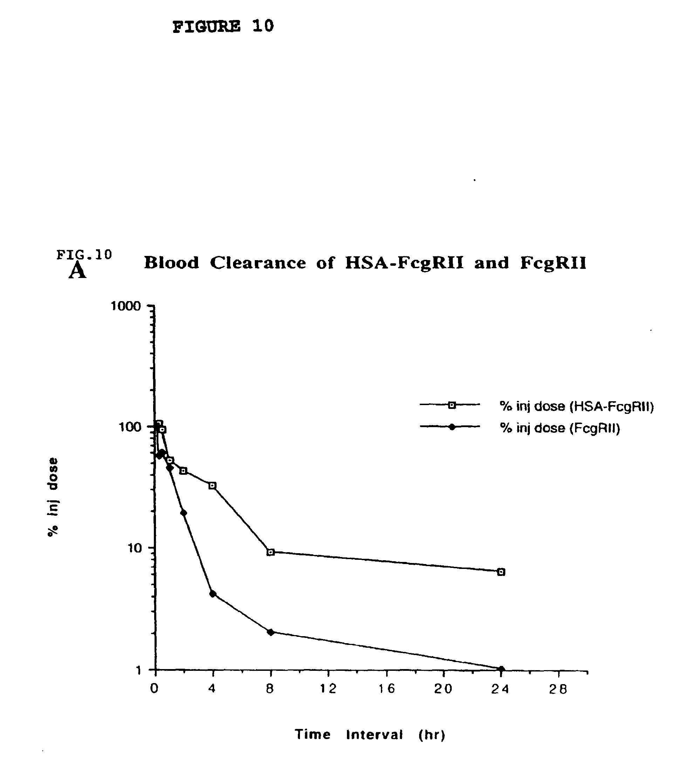

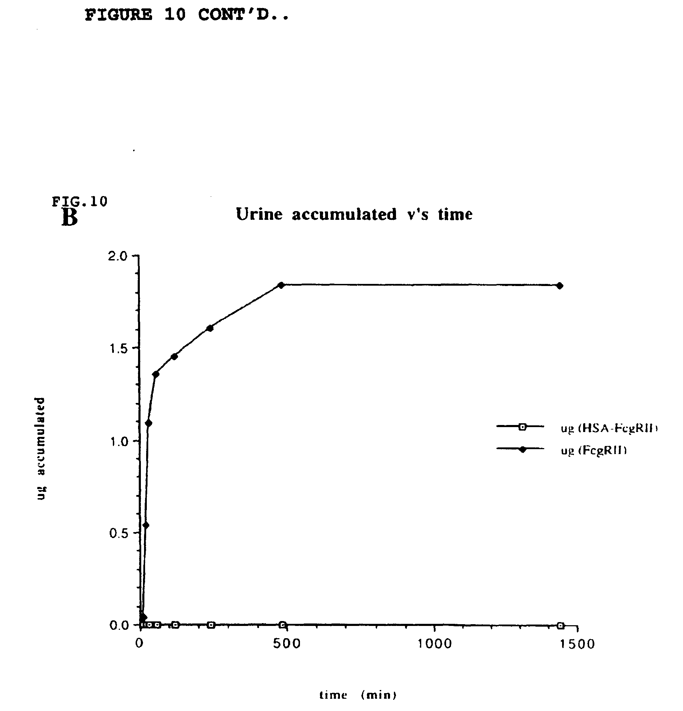

Figure 10a depicts curves showing the clearance of the HSA:FcγRII fusion protein from the blood of mice. The animals were injected with either HSA:FcγRII fusion protein or soluble FcγRII and the disappearance from the circulation measured. The conclusions are that the half life of the receptor FcγRII is approximately 40 minutes whilst that of HSA:FcγRII is 140 minutes. Also the HSA:FcγRII persists for many hours, eg. 9% of the dose was present after 8 hours and 7% at 24 hours. In contrast, all the soluble FcγRII was excreted.

The appearance of any receptor or fusion protein in urine was monitored (Fig. 10b). The soluble FcγRII appeared very rapidly whereas there was no detectable fusion protein in the urine, ie. this was not excreted.

### EXAMPLE 8 Method of Removing Immunoglobulin From a Sample

The FcR or a mutant or fusion protein thereof is attached to a solid support. When this method is used in plasmaphoroxis the solid support will be a membrane or the like. The substrate may be silica such as in this Example. Human albumin (native) and HSA:FcγRII were coupled to silica beads in two separate preparations.

The coupling of the protein to produce a reagent in accordance with the present invention may be achieved by standard methods. In the present Example the hydroxyl groups on the silica beads were replaced by amino groups via an exchange reaction with 3-aminopropyltriethyoxysilane (APTS). This activates the silica beads. The protein was mixed with a carbodimide (such as EDC) and added to the activated silica beads. Carboxyl groups on the protein combine with the carbodiimide to form an O-acylisourea derivative which in turn reacts with the amino groups on the silica beads to form an amide with elimination of the urea derivative.

In a different version of preparing the reagent, the protein and carbodiimide were reacted in the presence of N-hydroxysuccinimide to form a more stable amino-reactive intermediate. β-mercaptoethanol was added to quench the unreacted carbodiimide. The protein succinimide derivative was then added to the activated silica beads.

The preparation of human albumin attached to silica and HSA:FcγRII attached to silica was carried out as follows:
50 mg NH₂-silica beads
0.9 mg of HSA OR HSA-FcγRII
30 mg of EDC
in mPBS pH6 (4ml) incubated o/n at 4°C

**Table 2 In 2 parallel tubes with HSA (plus ¹²⁵I HSA)**

| | **3 washes mPBS** (pH 7.4) | **3 washes 0.1m NaHCO**_{**3**} (pH 8.3) |
|---|---|---|
| +EDC | 85% bound | 72% bound |
| -EDC | 30% bound | 14% bound |

Table 2 shows that after conjugation of HSA to silica (measured by the use of tracer labelled HSA in mixture), three washes with NaHCO₃ were required to remove non-specifically bound material.

### EXAMPLE 9 Ability oF HSA:FcγRII to Bind Immune Complexes

### MATERIALS & METHODS

Similar to Example 7. Each tube 2µg of silica matrix conjugated with either 1.8µg of the HSA:FcγRII fusion protein or HSA in a volume of 1ml of PBS and 0.5% BSA. Three hundred nanograms of either iodinated HAGG or monomeric IgG was added. Additional controls included tubes with no silica to determine non-specific depletion. Samples were taken at the indicated time points and the quantity of label HAGG or monomeric Ig remaining in the supernatant after removal of silica beads was determined.

In this Example the ability of one of the proteins of the present invention, HSA:FcγRII to bind immune complexes as represented by HAGG is illustrated.

The ability of the proteins of the present invention to bind one form of immunoglobulin and not another form has important therapeutic implications. The proteins of the present invention have been found to specifically bind immunoglobulin complex as opposed to monomeric Ig.

The binding of immune complexes in the form of HAGG was to HSA:FcγRII was tested. These results are described in Table 3.

**Table 3**

| Support | Tracer Ligand | % Ligand | Bound* |
|---|---|---|---|
| HSA-FcγRI | Hagg | 35.5 | 37.5 |
| RSA | Hagg | 4.0 | 5.4 |

| | | | |
|---|---|---|---|
| *bound after overnight at 4°C PBS+0.5 BSA | | | |

Table 3 describes the binding of immune complexes in the form of aggregated Ig (HAGG) to the BSA:FcγRIIa fusion protein but not to HSA on silica.

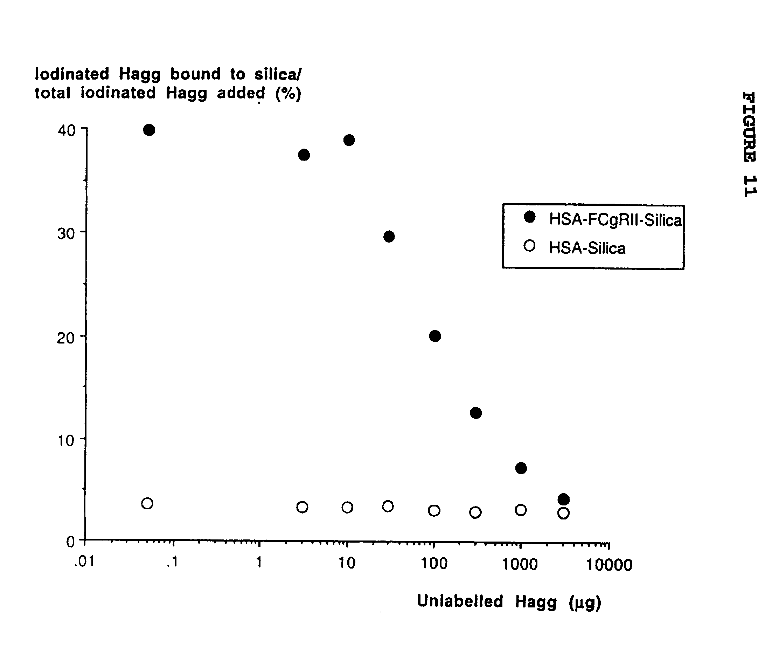

The interaction of HAGG with HSA:FcγRII was studied. This is shown in Figure 11.

The results shown in Figure 11 indicate that there is a clear difference in the binding of labelled HAGG to HSA:FcγRII compared to binding to HSA. In addition they show that as the concentration of unlabelled competitor HAGG is added, the binding of labelled HAGG is progressively inhibited to the level seen in HSA-silica.

### EXAMPLE 10 Immune Complex Assay

The polypeptides of the present invention are useful in detecting the presence of immune complexes. In this example it is clear that the polypeptides of the present invention can be used to detect immune complexes and that certain modifications may be made to optimise the assays.

Two approaches are used in the development of the immune complex assays. The first utilises recombinant soluble (rec.sFcγRII) in combination with anti-Fc receptor antibodies. The second approach uses the HSA fusion protein.

The assays described in this example use an ELISA format but the principles apply to any format such as chemiluminescence, biosensor, agglutination, etc.

### MATERIALS AND METHODS

### IMMUNE COMPLEX ASSAY (ICA)

### Coating Microtitre Stripwells with 8.26 Monoclonal Antibody

8.26 monoclonal antibody (mAb) diluted to 5 µg/ml in carbonate buffer pH 10.0. Aliquot 50 ul volumes of diluted 8.26 mAb were placed into Nunc maxisorp microtitre stripwells and incubated at 4°C for 12 hours. 8.26 mAb coated microtitre stripwells are stable for at least two weeks.

### Binding Capture of Recombinant FcRII

This step is performed just prior to assay. Stripwells were decanted prior to assay and wash x2 with PBS/0.2%BSA pH7.4. Wells are blocked by adding 200 µl PBS/2%BSA pH7.4 then incubated for 30 min at room temperature. This was decanted and 100 µl recombinant FcRII was added, 1µg/ml per well. This was then incubated at 37°C for 30 min. Wells were then washed x4 with PBS/0.2%BSA.

### Elisa Assay

Standard (100 µl), control or test sample were added per well. A standard curve was prepared by diluting heat aggregated IgG in PBS. This was incubated at 37°C for 30 min. then washed x4 with PBS/0.2%BSA. Working dilution of goat anti-human IgG alkaline phosphatase conjugate (100 µl) (Sigma) was added and then incubated at room temperature for on hour then washed x4 with PBS/0.2%BSA. p-nitrophenyl phosphate substrate (Sigma 104 phosphatase tablets:-2.5mg.ml carbonate buffer) (100 µl) was added and this was incubated at room temperature in the dark for 30 min. then the reading was taken at OD 405 nm. Note: Samples tested for anti-mouse reactivity by substituting mouse IgG2b (Sigma MOPC-141) for 8.26 mAb.

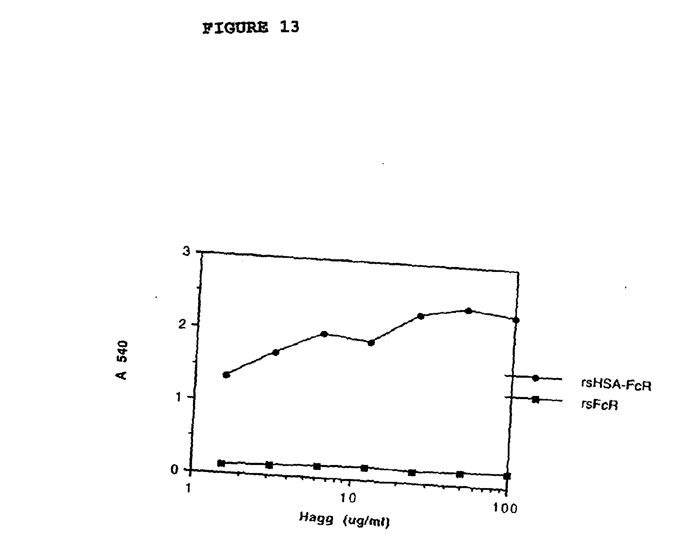

Fig. 13 shows that ELISA plates were coated with rec.sFcγRII denoted in the figure as rsFcγRII or with HSA:FcγRII fusion protein (denoted in the figure as rsHSA-FCR). Aggregated immunoglobulin (HAGG) was titrated and bound immunoglobulin detected using HRP conjugated anti-human Ig.

Using standard protocols for attachment of proteins to ELISA plates it is clear that "spacing" the protein with Fc binding ability away from the surface improves the activity of the protein. This can be accomplished by standard methods such as by using an antibody that binds to the polypeptide of the invention or by attaching the extracellular domains of the polypeptide to another molecule such as a protein, dextran, etc. A useful antibody for a spacer is antibody 8.2 or 8.26. The detection of immune complexes in the form of HAGG or in serum of patients with rheumatoid arthritis is improved by using these approaches. Table III below shows that rec.sFcγRII bound to anti-FcR antibody binds preferentially to HAGG over monomeric Ig (measured OD 405nm). The monomeric Ig is contaminated with 10-20% aggregates.

**Table 4 Monomeric V Heat Aggregated IgG (OD 405 nm)**

| Conc RFcRII ng/ml | RFcRII Only | RFcRII IgG* | RFcRII + HAGGG |
|---|---|---|---|
| 750 | 33 | 204 | 1230 |
| 500 | 23 | 204 | 1136 |
| 250 | 15 | 202 | 1444 |
| 125 | 18 | 158 | 1030 |
| 62.5 | 24 | 122 | 906 |
| 31.3 | 24 | 102 | 808 |
| Blank | 28 | 59 | 456 |

| | | | |
|---|---|---|---|
| * Mostly monomeric Ig (known contain 10-20% aggregated Ig) | | | |

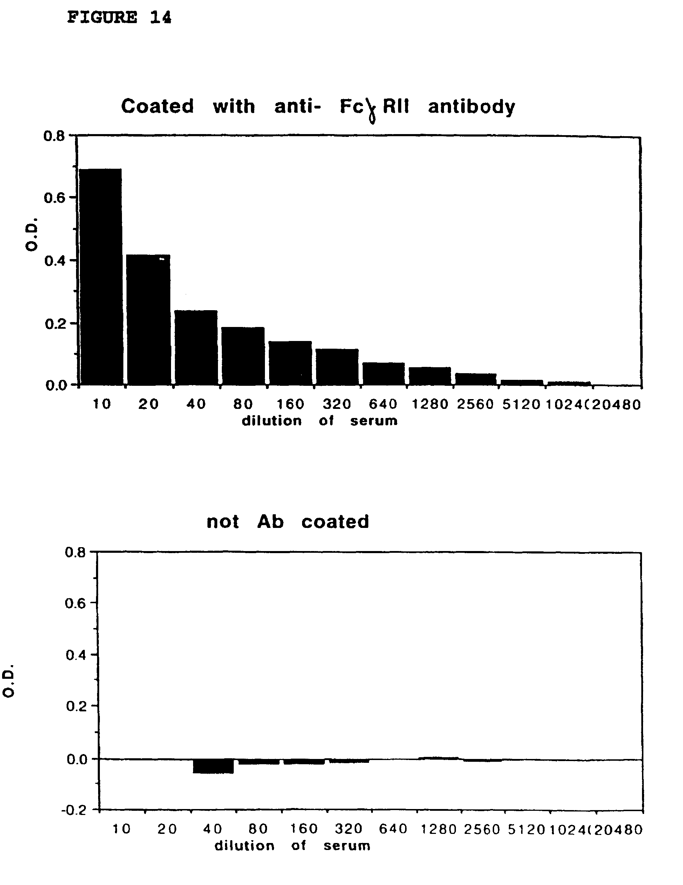

In this connection Fig 14 shown Elisa plates were either coated with anti-FCγRII antibody 8.2 and then with rec.sFcγRII (A) or with rec.sFcγRII (B). After blocking with BSA serum from a patient with rheumatoid arthritis was added and bound immunocomplexes resolved using HRP conjugated anti-human Ig. Using either of the above approaches immune complexes can be detected. Clearly variations on the above methods may be used.

### EXAMPLE 11 Immunophoresis Device & Methods of Use

Immune complexes may be removed from the circulation using the polypeptides of the present invention attached to a solid support in a plasmaphoresis device, for example. Attachment of such polypeptides to a solid support such as silica was discussed earlier in respect of HSA:FcγRII.

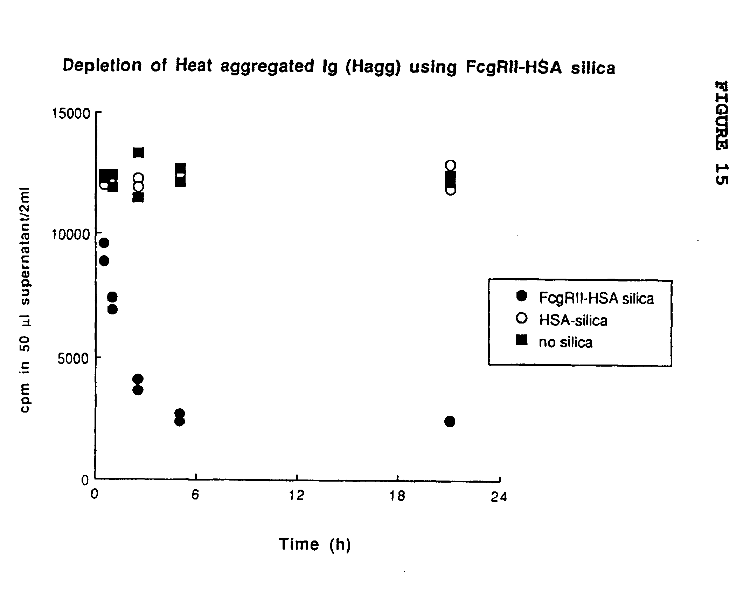

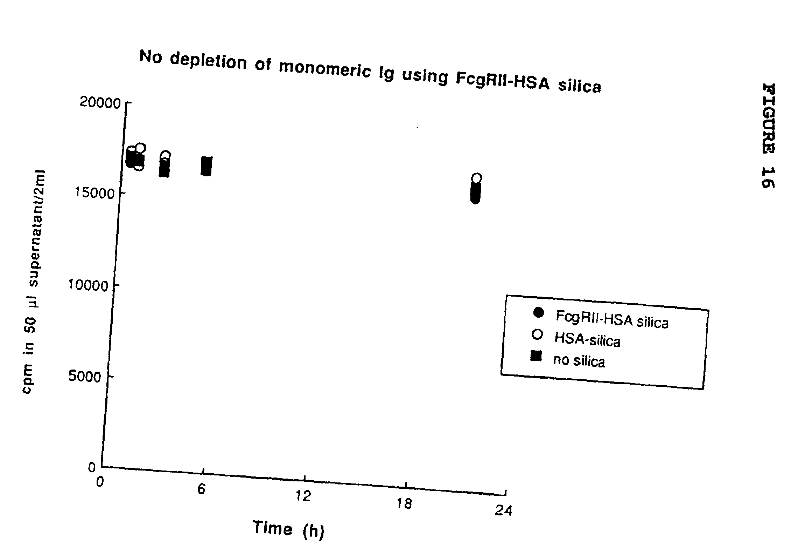

To demonstrate that the HSA:FcγRII protein is functional the following tests were conducted. It has been demonstrated that immune complexes (in the form of HAGG) bind to HSA:FcRII and not to HSA or HSA:silica. See Figures 11, 14, 15 and 16. Figure 14 demonstrates that ¹²⁵I labelled HAGG (A) or monomeric Ig (B) was incubated with HSA:FcγRII-silica or HSA silica for up to 20 hours at room temperature. Samples were removed at various time points and HAGG or Ig remaining after removal of the silica complexes was determined. Figure 15 shows immune complexes are depleted from liquid by incubation with HSA:FcγRII protein-silica resin but not by HSA-silica resin. We have also demonstrated that monomeric immunoglobulin does not bind to HSA-FcγRII. In this connection see Fig 16 in which monomeric radio-labelled immunoglobulin does not bind to HSA-FcγRII silica or to HSA-silica.

### EXAMPLE 12 Assay for the Discovery of Antagonists to Fc Receptors

A cell free system has been devised to detect the presence of compounds that inhibit the activity of Fc receptors. In the present example FcγRII is exemplified but the strategy is equally applicable to other Fc receptors.

The principle of the invention is to use known or unknown compounds to attempt to inhibit the binding of polypeptides with Fc binding ability to immune complexes. The polypeptides of the present invention may be labelled directly or indirectly. These polypeptides may be rec.sFcγRII or HSA:FcRII. In the format described, an ELISA assay is used wherein immune complexes are attached to a surface under standard incubation conditions. Polypeptides of the invention are directly labelled with a reporting enzyme, horseradish peroxidase (HRP) and mixed with the putative antagonists. This mixture is added to the immune complexes and any inhibition of binding of the polypeptides of the invention to immune complexes results in a decrease in the colour development when HRP-substrate is added as per a standard ELISA.

It is important to note that any reporting substance could be used, eg. radioiodine, other enzymes such as alkaline phosphatase, beads or erythrocyte to which the receptor had been attached, flurogenic substances, etc. In the present format HRP is attached to HSA-FcγRII. Our results indicated that when HRP is used as a label it is necessary to employ a spacer in order to preserve Fc binding ability.

An alternative strategy which requires use of an indirect assay may be employed. In such an assay the polypeptide of the invention is added to a mixture of putative inhibitors and the mixture is then added to the immune complexes which are attached to a surface. Following an incubation period the surface is washed and any Fc receptor that is bound is detected using antireceptor antibody. Any antagonists of the Fc binding ability of the polypeptides inhibit the binding to immune complexes and reduce potential signal.

Specifically the following protocol was used. HAGG plates were prepared by incubating 50 µl-well HAGG at 25 µg/ml in coating buffer (0.5M carbonate/bicarbonate, pH9.6), for 16 hours at 37°. Some wells contained no HAAG as a negative control. To prevent non-specific binding to the surface, the wells of the plates were treated with mPBS containing 1% (w/v) bovine serum albumin at 3 hours at 37°. The wells were then rinsed three times by immersion in mPBS. Samples containing soluble FcγRII were diluted as required and added in a volume of 30 µl. Dilutions of a standard of rec.sFcγRII were made and added in a volume of 30 µl to generate a standard curve. Last, 30 µl of a detection reagent (consisting of a 1/1000 dilution of Amersham (#9310) anti-mouse IgFab'2 fragment HRP conjugate and the anti-FcγRII antibody 8.2 at 0.6 µg/µl (in PBS, 1% BSA) was added and incubated for 1 hour at 37°. The plates were then washed by immersion 8 times in PBST. Subsequently ABTS reagent was added (100 ml) and read at a 405 nm.

The data in Figure 17 demonstrates that rec.sFcγRII produced in mammalian cells (CMOrsFcR) or in bacteria (C2rsFcγR2) or even as a fusion protein with a bacterial maltose binding protein (C2MBPrsFcR) can be used in this assay. The specificity of the assay is demonstrated by the fact that the molecule comprising a single FcγRII domain (d2) shows no detectable binding to immune complexes.

It should be noted that in both examples it is possible that other receptors could be used to detect binding to other immunoglobulin classes and immune complexes such as IgE and FcεR, IgM and FcµR or FcγRIII or FcγRII and IgG complexes.

We have been able to demonstrate that the above method and variations thereof are able to usefully detect compound that inhibit the interaction of FcγRII with immune complexes.

Specifically the following method has been used. HAGG plates were prepared by incubating 50 µl/well at 25 µg/ml in coating buffer for 16 hours at 37°C. A negative control containing no HAAG was also prepared. To prevent non-specific binding to the surface, the wells of the plates were treated with mPBS containing .5% (w/v) Tween 20 (for library screening) or 1% (w/v) bovine serum albumin for 3 hours at 37°C.

The specificity tests for inhibition of binding of rec.sFcγRII were conducted as follows. Wells were rinsed 3 times by immersion in mPBS containing 0.05% (w/v) Tween 20 (PBST). Serial 1:2 dilutions in a volume of 25 µl of rec.sFcγRII (purified from supernatants of CHO cells transfected with DNA encoding rec.sFcRII) with starting concentration of 45 µg per µl. The HRP conjugated HSA:FcγRII fusion protein was added (25 µl at 1:100 dilution). This corresponds to approximately 4 µg/µl or 40nM wrt rsHSA-FcγRII fusion HRP-conjugate. This was incubated for 1 hour at 37°C.

Screening of the libraries of compounds or biological preparations the inhibition of Fc binding activity were conducted as follows. Wells were rinsed three times by immmersion in mPBS containing .05% w/v Tween 20 (PBST). Solutions (2 µl) containing compounds were added to a solution containing HRP conjugated HSA:FcγRII fusion protein (100 µl at 1:200 dilution). This corresponds to approximately 4 µg/µl or 40 nM wrt rsHSA-FcγRII fusion HRP-conjugate). This was incubated for 1 hour at 37°C then 90 µl was transferred to a plate containing HAGG as defined above.

Screening biological preparations or libraries for inhibition of Fc binding ability (second version). Wells were rinsed by immersion of PBS as described above. Serial dilutions of the solution (patients sera or libraries of compounds) in 100 µl of mPBS containing 1.0% BSA (w/v) were incubated for 1 hour at 37°C, plates were washed 4 times in PBS and then the HRP conjugated HSA:FcγRII fusion protein was added (50 µl at 1:100 dilution). This corresponds to approximately 8 µg/µl or 80 nM wrt rsHSA:FcγRII fusion protein HRP-conjugate. This was incubated for one hour at 37°C. The plates were then washed by 5 times immersion in PBST. ABTS reagent (100 µl) was added and this was read at A405nm.

Data (not shown) was generated by using rec.FcγRII to specifically inhibit the binding of HRP conjugated HSA:FcγRII protein to immune complexes that were attached to the plate surface. As increasing quantities of the rec.sFcRII were added to the assay the amount of HRP-HSA:FcγRII binding to HAGG decreased. These results indicated that the binding of the HRP-HSA:FcγRII fusion protein to immune complexes is specific and that inhibitors of the interactions between FcγRII and immune complexes can be detected.

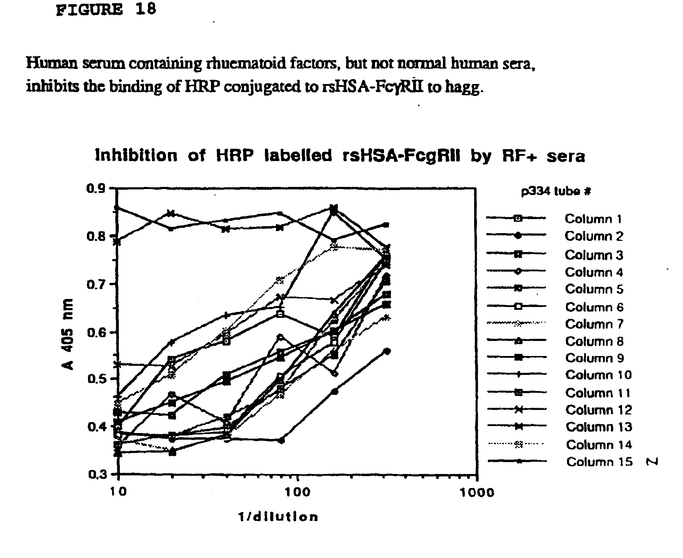

In another experiment (Fig 18) the presence of inhibitors of FcγRII:immune complex interactions were identified in the sera of patients. In these cases the sera were titrated in the HAGG coated elisa plates and HRP-HSA:FcγRII fusion protein was added. Clearly the sera of the patients containing rheumatoid factors inhibit the binding of the HRP-HSA:FcγRII protein to immune complexes. This is indicated by a reduction in the absorbence compared to that obtained using normal sera (column 15).

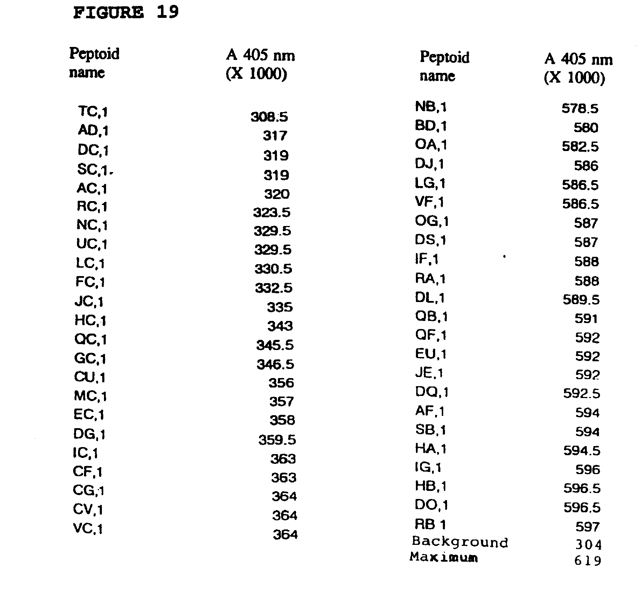

In another experiment the HRP-HSA:FcγRII was used to screen a library of organic compounds. This library was produced by standard chemistry as described in Simon et al PNAS 89 :9367 (1992) "Peptoids A Modular Approach to Drug Discovery". In this a collection of synthesised compounds is produced and the capacity of individual compounds (or sets of compounds) to inhibit the binding of HRP-HSA:FcγRII to immunogoblin is assessed by preincubating the library components (the compounds) with HRP-HSA:FcγRII fusion protein or any other polypeptide of the present invention. The polypeptides may be specific for other classes of immunoglobulin such as IgE or IgA. The HRP-HSA:FcγRII is mixed with the compounds and the effect on binding to HAGG (or any immune complex) is determined as described directly above. Inhibition of binding is indicated by decreased absorbence compared to the control (no inhibitor). The results of such an experiment are given in Fig 19. This shows a di-peptoid library which was screened as described above. As an example of the type of results obtained, the maximum binding is indicated by an absorbance (at 540 nm) of .619 units. In the presence of compound TC1 this absorbance value falls to .3085 which is equivalent to the background value, .304. TC1 completely inhibits the interaction with immune complexes of the HRP polypeptide conjugate. In addition some compounds clearly do not inhibit the interaction, for example RB1. Incubation of the conjugate with RB1 does not inhibit the interaction since an absorbance of .597 was obtained which is similar to the maximum binding (.619) obtained by the conjugate binding to HAGG in the absence of any inhibitor. It should be noted that these functional assays can be used to screen for inhibitors of Fc receptor function either by binding to the Fc receptor or by binding to the immune complex (IgA, IgE, IgG, IgM, IgD) at the site where the Fc receptors bind. This second type of antagonist does not fit the classical definition of an antagonist of Fc receptor function, however, it is still within the scope of the present invention in as far as the present invention relates to a method of testing compounds for their ability to inhibit Fc receptor function and to the antagonists per se identified by such a method.

### REFERENCES

1. Unkeless, J.C., & Freedman V.H.
   Ann. Rev. Immunol., 1988 6 251-281.
2. Van de Winkel, J.G.J. & Anderson, C.L. J. Leuk. Biol., 1991 49 511-524.
3. Ravetch, J.V. & Kinet, J.-P.
   Ann. Rev. Immunol., 1991 9 457-492.
4. Graziano, R.F. & Fanger, M.W. J. Immunol., 1987 139 3536-3541.
5. Anderson, C.L., Shen, L., Eicher, D.M., Wewers, M.D. & Gill, J. K.
   J. Exp. Med., 1990 171 1333-1345.
6. Rigley, K.P. & Klaus, G.B.
   Eur. J. Immunol., 1989 19 481
7. Stuart, S.G., Troustine, M., Vaux, T., Koch, T., Martens, C., Hellman, I. & Moore, K. W.
   J. Exp. Med., 1988 166 1668-1684.
8. Hibbs, M.L., Bonadonna, L. Scott, B.M.,
   McKenzie, I.F.C. & Hogarth, P.M.
   Proc. Natl. Acad. Sci. (USA), 1988 85 2240-2244.
9. Stengelin, S., Stamankovic, I. & Seed, B.
   EMBO J., 1988 7 1053-1059.
10. Brooks, D.G., Qiu, W.Q., Luster, A.D. & Ravetch, J.V.
   J. Exp. Med., 1989 170 1369-1385.
11. Stuart, S.G., Simister, N.E., Clarkson, S.B.,
   Shapino, M. & Mellman, I.
   EMBO J., 1989 3657-3666.
12. Allen, J.M. & B. Seed.
   Science, 1989 243 378-381.
13. Simmons, D. & Seed B.
   Nature, 1988 333 568-570.
14. Ravetch, J.V. & Perussia, B.
   J. Exp. Med., 1989 170 481-491.
15. Shimizu, A., Tepler, I., Bemfrey, P.N.,
   Berenstein, E.H. and Leder P.
   Proc. Natl. Acad. Sci. (USA), 1988 85 1907-1911.
16. Maliszewski, C.R., March, C.J., Shoenborn, M.A., Gimpel, S. & Li, S.
   J. Exp. Med., 1990 172 1665-1672.
17. Hulett, M.D., McKenzie, I.F.C. & Hogarth, P.M. Bur. J. Immunol., 1993 23 640-645.
18. Hogarth, P.M., Hulett, M.D., Ierino, F.L. Tate, B., Powell, M.S. & Brinkworth, R.I.
   Immunol. Rev., 1992 125 21-35.
19. Ierino, F.L., Hulett, M.D., McKenzie, I.F.C. & Hogarth, P. M.
   J. Immunol., 1993 150 1794-1799.
20. Hulett, M.D., Witort, E., Brinkworth, R.I., McKenzie, I.F.C. & Hogarth, P.M.
   J. Biol. Chem., 1994 269 15287-15293.
21. Tax, W.J.M., Willems, H.W., Reekers, R.W. Capel, P.J.A. & Koene R.A.P.
   Nature, 1983 304 445-447.
22. Warmerdam, P.A., Van de Winkel, J.G.J., Vlug, A., Westerdaal, N.A.L. & Capel, P. J. A.
   J. Immunol., 1992 147 1338-1343.
23. Tate, B.J., Witort, B., McKenzie, I.F.C. & Hogarth, P.M.
   Immunol. and Cell Biol., 1992 70 79-87.
24. Hibbs, M.L., Hogarth, P.M. & McKenzie, I.F.C. (1985) Immunogenetics, 1985 22 335-348.
25. Lah, M., Quelch, K., Deacon, N.J., McKenzie, I.F.C. & Hogarth, P.M.
   Immunogenetics, 1990 31 202-206.
26. Robinson, M.W.
   J. Biol. Chem., 1993 268 12736-12743.
27. Horton, R.M., Hunt, H.D., Ho, S.M., Pullin, J.K. & Pearse, L.R.
   Gene, 1988 77 61-68.
28. Sambrook, J., Fritsch, E.F., and Maniatis, T. (1989) Molecular cloning: A Laboratory Manual, 2nd Ed., Cold Spring Harbor Laboratory, Cold Spring Harbor, NY.
29. Van Doren, K., Hanahan, D., & Gluzman, Y
   J. Virol., 1984 50 606-614 .
30. Sanger, F., Nicklen, S., and Coulson, A.R. (1977) Proc. Natl. Acad. Sci. (USA), 1977 74 5463-5467.
31. Kraft, R., Tardiff, J., Krauter, K.S. and
   Leinward, L.A.
   Biotechniques, 1988 6, 544-547.
32. Seed, B., and Aruffo, A.
   Proc. Natl. Acad. Sci. (USA), 1987 84 3365.
33. Lopez, A.F., Strath, M. and Sanderson, C.J.
   Immunology, 1983 48 503-509.
34. By, P.L., Prowse, S.J. and Jenkins, C.R.
   Immunochemistry, 1978 15 429-435.
35. Pietersz, G.A., Kanellos, J. & McKenzie, I.F.C.
   Cancer Res., 48 4469-4476.
36. Parish, C.R. and Hayward, J.A.
   Proc. R. Soc. Lond. (Biol.), 1974 187 47-56.
37. Harlow, E. and Lane, D. (1988)
   Labelling antibodies. In Antibodies - A Laboratory
   Manual. Cold Spring Harbor Laboratories. Cold Spring Harbor, NY.
38. Farber, D.L., Giorda, R., Nettleton, M.Y., Trucco, M., Kochan, J.P. & Sears, D.W.
   J. Immunol., 1993 150 4364-4361.
39. Hibbs, M.L., Tolvanen, M. & Carpen, O. J. Immunol., 1994 152 4466-4474.
40. Mallimaci, M.A., Chizzonite, R., Griffen, M.,
   Nettleton, M., Hakimi, J., Tsien, W-H. & Kochan, J. P.
   J. Biol. Chem., 1993 268 22079-22083.
41. Riske, F., Hakimi, J., Mallamaci, M., Griffen, M.,
   Pilson, B., Tobkes, N., Lin, P., Danho, W., Kochan, J.
   & Chizzonite, R.
   J. Biol. Chem., 1991 266 11245-11251.

The invention will now be described by way of a series of numbered clauses.
1. An isolated polypeptide with Fc binding ability, wherein the polypeptide is altered compared to a native Fc receptor by addition, deletion and/or substitution of one or more amino acids compared to said native receptor and said alteration results in improved characteristics compared to said native receptor.
2. The polypeptide of clause 1 which is an Fc receptor-like molecule with an altered ability to bind Fc wherein said altered ability is brought about by alteration of one or more amino acids which affect immunoglobulin binding ability.
3. The polypeptide of clause 1 or clause 2 which is able to bind IgG, IgE, IgA, IgM or IgD.
4. The polypeptide of clause 3 wherein the alterations are in the first and/or second domain.
5. The polypeptide of clause 4 wherein the alterations are in A/B, C/C' and/or E/F loops of domain 1 and/or G/A strand and where the polypeptide is able to bind IgG or IgE.
6. The polypeptide of clause 4 wherein the alterations are in B/C, C'/E, and/or F/G loops of domain 2 and where the polypeptide is able to bind IgG or IgE.
7. The polypeptide of clause 5 or clause 6 having enhanced ability to bind Fc compared to a native Fc receptor in a given class.
8. The polypeptide of clause 7 wherein the alterations are in the first domain at amino acid positions 133, 134, 158, 159 and/or 160 compared to a native Fc receptor.
9. The polypeptide of clause 8 wherein the amino acids are changed to alanine.
10. The polypeptide of clause 9 wherein Asp¹³³ and/or Pro¹³⁴ are changed to alanine.
11. The polypeptide of clause 7 wherein the alterations are in the second domain at amino acid positions 130, 156, 160 and/or 161 compared to a native Fc receptor.
12. The polypeptide of clause 11 wherein the amino acids altered are changed to alamine.
13. The polypeptide of clause 12 wherein Trp¹³⁰, Trp¹⁵⁶, Tyr¹⁶⁰ and/or Glu ¹⁶¹ are changed to alanine.
14. The polypeptide of clause 4 having a reduced ability to bind immunoglobulin compared to a native Fc receptor in a given class.
15. The polypeptide of clause 1 wherein the polypeptide is altered compared to a native Fc receptor such that the size of the polypeptide is larger than said native Fc receptor.
16. The polypeptide of any one of clauses 1 to 15 which is soluble.
17. The polypeptide of clause 15 or clause 16 wherein said size is 67 to 1000 kD.
18. The polypeptide of clause 15 in the form of a fusion protein comprising an Fc binding component and a fusion component.
19. The polypeptide of clause 18 wherein the Fc binding component is an extracellular region of a native Fc receptor, a portion of said native Fc receptor or the polypeptide of any one of clauses 1 to 7.
20. The polypeptide of Clause 19 wherein the fusion component is a physiologically tolerated protein or other molecule selected from the group of human serum albumin, Fc receptor, complement regulating molecules, complement receptors, cytokine receptors, dextran, carbolydrate, polyetheylene glycol and synthetic polymers.
21. The polypeptide of clause 20 comprising HSA:FcγRII.
22. The polypeptide of clause 1 comprising a component capable of detection.
23. The polypeptide of clause 22 wherein the component capable of detection comprises a direct or indirect label.
24. The polypeptide of clause 23 wherein said component capable of detection is a radiolabel, chemiluminescent label, chromophoric label, a labelled antibody, or a detectable enzyme such as horse radish peroxidase or alkaline phosphatase.
25. A method of testing a compound for its ability to act as an antagonist of an Fc receptor said method comprising contacting the compound with a polypeptide with Fc binding ability or a Fc receptor or part thereof under conditions and for a time sufficient to allow any binding of the compound and the polypeptide or Fc receptor or part thereof to take place, and then determining whether binding has occurred.
26. The method of clause 25 wherein the polypeptide of any one of clauses 1 to 7, 14, 15 and 22.
27. The method of clause 25 wherein said compound is tested for its ability to inhibit binding of IgG or IgE.
28. An antagonist compound isolated by the method of clause 26.
29. The antagonist of clause 28 wherein said compound is capable of blocking the function of the A/B, C/C', and/or B/F loops in the first domain and/or the B/C, C'/E and/or F/G loops in the second domain and/or the G/A strand of an IgG or an IgE Fc receptor.
30. The antagonist of clause 28 which acts by binding Ig rather than Fc receptor.
31. A pharmaceutical composition comprising the polypeptide of any one of clauses 1 to 21 or the antagonist of clause 28 together with a pharmaceutically appropriate carrier or diluent.
32. An isolated nucleic acid molecule encoding a polypeptide with Fc binding ability wherein the polypeptide is altered compared to a native Fc receptor by addition, deletion and/or substitution of the amino acids encoded by the nucleic acid compared to a native Fc receptor and wherein said alteration results in improved characteristics compared to said native receptor.
33. The molecule of clause 32 which encodes an Fc receptor-like molecule comprising an altered ability to bind immunoglobulin wherein said ability is brought about by alteration of one or more amino acid residues which affect immunoglobulin binding ability.
34. The molecule of clause 32 or clause 33 wherein said polypeptide encoded binds IgG, IgE, IgA, IgM or IgD.
35. The molecule of clause 32 which encodes a soluble polypeptide.
36. The molecule of clause 33 wherein the polypeptide encoded has alterations in A/B, C/C' and/or B/F loops of domain 1 and/or G/A strand and where the polypeptide is able to bind IgG or IgE.
37. The molecule of clause 33 wherein the polypeptide encoded has alterations in the B/C, C' /E and/or F/G loops of domain 2 and where the polypeptide is able to bind IgG or IgE.
38. The molecule of clause 33 which encodes a polypeptide with an enhanced ability to bind immunoglobulin compared to a native Fc receptor in a given class.
39. The molecule of clause 36 wherein the alterations are in the first domain at amino acid positions 133, 134, 158, 159 and/or 160 compared to a native Fc receptor.
40. The molecule of clause 39 wherein the amino acid altered are changed to alanine.
41. The molecule of clause 40 wherein Asp¹³³ and/or Pro¹³⁴ are changed to alanine.
42. The molecule of clause 37 wherein the alterations are in the second domain at amino acid positions 130, 156, 160 and/or 161.
43. The molecule of clause 42 where in the amino acids altered are changed to alanine.
44. The molecule of clause 43 wherein Trp¹³⁰, Trp¹⁵⁶, Tyr¹⁶⁰ and/or Glu¹⁶¹ are changed ot alanine.
45. A molecule of clause 33 encoding a polypeptide with a reduced ability to bind immunoglobulin compared to a native Fc receptor.
46. The molecule of clause 32 wherein the polypeptide is altered compared to a native Fc receptor such that the size of the polypeptide is larger than said native Fc receptor.
47. The molecule of any one of clauses 32 to 46 wherein the polypeptide is soluble.
48. The molecule of clause 46 or clause 47 wherein the size of the polypeptide is 67 to 1000 kD
49. The molecule of clause 46 wherein said polypeptide is a fusion protein comprising an Fc binding component and a fusion component.
50. The molecule of clause 49 wherein said Fc binding component is provided by a nucleotide sequence which encodes a polypeptide having enhanced Fc binding ability compared to a native Fc receptor or by a nucleotide sequence encoding a native Fc receptor or a portion thereof.
51. The molecule of clause 50 wherein the fusion component encoded is a physiologically tolerated protein selected from the group of human serum albumin, Fc receptors, complement receptors, complement regulating proteins and cytokine receptors.
52. The molecule of clause 51 being the construct encoding HSA:FcγRII.
53. A vector comprising the molecule of any one of clauses 32 to 52.
54. A host cell transformed or transfected with the vector of clause 53.
55. A method of making a nucleic acid molecule encoding a polypeptide with Fc binding ability wherein the polypeptide is altered compared to a native Fc receptor by addition, deletion and/or substitution of one or more amino acids encoded by said molecule compared to a native Fc receptor and wherein said alteration results in improved characteristics compared to said native receptor, said method comprising producing a nucleic acid molecule encoding the polypeptide by addition, deletion and/or substitution of one or more codons specific for amino acids affecting immunoglobulin binding ability of the polypeptide.
56. A method of producing the polypeptides of any one of clauses 1 to 24 comprising obtaining expression of the polypeptide in the host cell of clause 54.
57. A method of determining the presence and/or amount of immunoglobulin in a sample said method comprising contacting said sample with the polypeptide of any one of clauses 1 to 24 or an Fc receptor or part thereof, for a time and under conditions sufficient to allow the polypeptide or the Fc receptor or part thereof and any immunoglobulin present in said sample to bind, and detecting presence of and/or determining the amount of said bound polypeptide - immunoglobulin, bound Fc receptor-immunoglobulin or bound part Fc receptor-immunoglobulin.
58. A kit for detecting immunoglobulin, including for detecting immune complexes, in a sample, said kit comprising in compartmentalised form, a first compartment adapted to receive the polypeptide of any one of clauses 1 to 24
   or an Fc receptor or a part thereof and at least one other compartment adapted to contain a detector means.
59. The method of clause 57 or the kit of clause 58 wherein said polypeptide is specific for IgG or IgE.
60. A method of removing immunoglobulin from a sample comprising contacting said sample with the polypeptide of any one of clauses 1 to 21 or an Fc receptor or part thereof for a time and under conditions sufficient for any immunoglobulin present in the sample to form a complex with said polypeptide or Fc receptor or part thereof and separating said complex from the remainder of the sample.
61. A method of removing immunoglobulin from a body fluid comprising taking body fluid from a patient, contacting the body fluid with the polypeptide of any one of clauses 1 to 21 or an Fc receptor or part thereof, for a time and under conditions sufficient to allow the polypeptide, Fc receptor or part thereof to bind said immunoglobulin, removing said bound immunoglobulin from the body fluid and replacing said body fluid in the patient.
62. The method of clause 60 or clause 61 wherein said immunoglobulin removed is immunoglobulin complex.
63. The method of clause 62 wherein the polypeptide used is specific for IgG or IgE.
64. A method of treatment of disease where an excess of immunoglobulin is implicated as a causative agent of the disease said method comprising administering an effective amount of the polypeptide according to any one of clauses 1 to 21 to said patient.

## Claims

1. An isolated polypeptide with Fe binding ability, wherein the polypeptide comprises an Fc binding component comprising an extracellular region of a native FcγRII receptor and a fusion component.

2. The polypeptide of claim 1, wherein the fusion component is selected from the group consisting of an immunoglobulin, human scrum albumin (HSA), Fc receptor, complement receptor, cytokine receptor, dextran, carbohydrate and polyethylene glycol.

3. The polypeptide of claim 2, wherein the fusion component is an immunoglobulin.

4. The polypeptide of claim 2, wherein the polypeptide is HSA:FcγRII.

5. The polypeptide of any one of claims 1 to 4 which is soluble.

6. A pharmaceutical composition comprising a polypeptide according to any one of claims 1 to 5 together with a pharmaceutically appropriate carrier or diluent.

7. The use of a polypeptide according to any one of claims 1 to 5 in the manufacture of a medicament for treating a disease where an excess of immunoglobulin is implicated as the causative agent of the disease.

8. The use of claim 7, wherein the disease is rheumatoid arthritis.

9. A method of removing immunoglobulin from a body fluid, said method comprising combining an effective amount of the polypeptide according to any one of claims 1 to 5 with the body fluid.

10. An isolated polypeptide with Fc binding ability, wherein the polypeptide is altered compared to a native Fc receptor by addition, deletion and/or substitution of one or more amino acids compared to said native receptor and said alternation results in improved characteristics compared to said native receptor with the proviso that where the polypeptide is able to bind IgG and a single alteration is present, the alteration is at a position other than residues 154 to 161 of domain 2.
